(19) Europäisches Patentamt / European Patent Office / Office européen des brevets

(11) EP 4 036 136 A1

(12) EUROPÄISCHE PATENTANMELDUNG

(43) Veröffentlichungstag:
**03.08.2022 Patentblatt 2022/31**

(21) Anmeldenummer: **21153995.2**

(22) Anmeldetag: **28.01.2021**

(51) Internationale Patentklassifikation (IPC):
***C08G 18/24*** $^{(2006.01)}$ ***C08G 18/48*** $^{(2006.01)}$
***C08G 18/73*** $^{(2006.01)}$ ***C08G 18/32*** $^{(2006.01)}$
***C08G 18/10*** $^{(2006.01)}$ ***A61F 2/00*** $^{(2006.01)}$
***C08L 75/02*** $^{(2006.01)}$ ***A61F 2/06*** $^{(2013.01)}$

(52) Gemeinsame Patentklassifikation (CPC):
(C-Sets verfügbar)
**C08G 18/4854; A61F 2/06; C08G 18/10; C08G 18/244; C08G 18/3215; C08G 18/3228; C08G 18/325; C08G 18/73; C08L 75/02;**
A61F 2240/001 (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder:
- **Medizinische Universität Wien**
  **1090 Wien (AT)**
- **Technische Universität Wien**
  **1040 Wien (AT)**
- **Ludwig Boltzmann Gesellschaft - österreichische Vereinigung zur Förderung der wissenschaftlichen Forschung**
  **1090 Wien (AT)**

(72) Erfinder:
- **Baudis, Stefan**
  **1230 Wien (AT)**
- **Bergmeister, Helga**
  **1030 Wien (AT)**
- **Ehrmann, Katharina**
  **1090 Wien (AT)**
- **Grasl, Christian**
  **1210 Wien (AT)**
- **Liska, Robert**
  **2123 Schleinbach (AT)**
- **Podesser, Bruno**
  **1220 Wien (AT)**
- **Schima, Heinrich**
  **1020 Wien (AT)**

(74) Vertreter: **Ellmeyer, Wolfgang**
**Häupl & Ellmeyer KG**
**Patentanwaltskanzlei**
**Mariahilfer Strasse 50**
**1070 Wien (AT)**

# (54) VERFAHREN ZUR HERSTELLUNG VON GEFÄSSPROTHESEN

(57) Die Erfindung betrifft die Verwendung eines thermoplastischen Poly(urethan-harnstoff)-Polyaddukts mit sterisch gehinderten Harnstoffgruppen der nachstehenden Formel (I) in einem Elektrospinn-Verfahren zur Herstellung von Gefäßprothesen:

$$-[I-M-(I-C_1)_a-(I-M)_b-(I-C_2)_c]_n- \qquad (I)$$

worin I, M, $C_1$ und $C_2$ jeweils für zweiwertige Reste stehen, die jeweils über eine Urethan- oder eine Harnstoffgruppierung miteinander verbunden sind, wovon
I jeweils unabhängig für einen zweiwertigen, von einem Diisocyanat abgeleiteten Rest mit 1 bis 20 Kohlenstoffatomen steht;
M jeweils unabhängig für einen zweiwertigen, von einem Makrodiol abgeleiteten Rest eines aliphatischen Polyethers, Polyesters oder Polycarbonats mit einem zahlenmittleren Molekulargewicht $M_n \geq 500$ steht;
$C_1$ jeweils unabhängig für einen zweiwertigen, von einem Diamin oder Aminoalkohol mit jeweils zumindest einer sterisch gehinderten sekundären Aminogruppe abgeleiteten Rest mit 1 bis 30 Kohlenstoffatomen steht;
$C_2$ jeweils unabhängig für einen zweiwertigen, von einem Diol, Diamin oder Aminoalkohol ohne sterisch gehinderte sekundäre Aminogruppen abgeleiteten Rest mit 1 bis 20 Kohlenstoffatomen steht;
wobei zumindest einer der Reste I, M, $C_1$ und $C_2$ eine oder mehrere Estergruppierungen umfasst; und

EP 4 036 136 A1

a, b und c jeweils unabhängig für eine ganze Zahl von 0 bis 10 stehen und n eine Zahl $\geq 3$ ist, die für die Anzahl der Blöcke des Polyaddukts steht;
mit der Maßgabe, dass jeweils innerhalb desselben Blocks $a + c \geq 1$ ist und in allen Blöcken zusammen zumindest ein $a \geq 1$ ist und zumindest ein $c \geq 1$ ist.

**Beispiel 2**

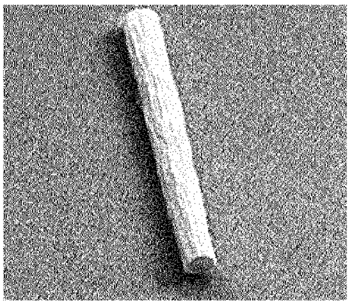

**Fig. 2a**
(außen)

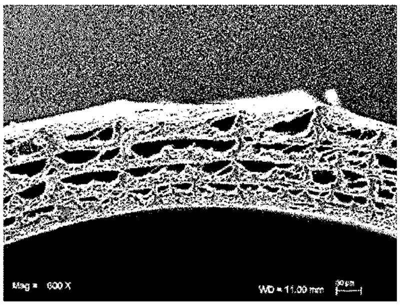


**Fig. 2b**
(Querschnitt)

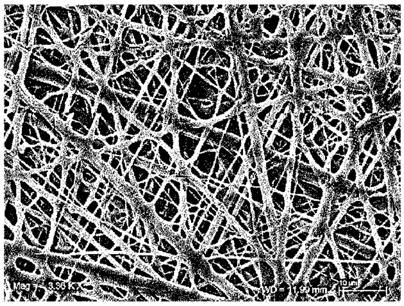

**Fig. 2c**
(innen)

## Figur 2

(52) Gemeinsame Patentklassifikation (CPC): (Forts.)

C-Sets
**C08G 18/10, C08G 18/3215;**
**C08G 18/10, C08G 18/3228;**
**C08G 18/10, C08G 18/325**

**Beschreibung**

STAND DER TECHNIK

Weltweit sind viele Patienten von Herz-Kreislauf-Erkrankungen betroffen. Der Verschluss von Gefäßen mit mittlerem und kleinem Durchmesser ist die häufigste pathologische Ursache von Herz-Kreislauf-Erkrankungen. Trotz bedeutender Fortschritte bei interventionellen Revaskularisierungsverfahren ist die chirurgische Therapie zur Behandlung von verschlossenen Blutgefäßen häufig indiziert. Aufgrund der vorteilhaften biomechanischen Eigenschaften und der geringen Thrombogenität sind autologe Blutgefäße das bevorzugte Ersatzmaterial für die Rekonstruktion von Blutgefäßen mit kleinem Durchmesser. Bei vielen Patienten scheitert dieser Therapieansatz jedoch aufgrund unzureichender Gefäßqualität des autologen Transplantates. Prothetische Gefäßprothesen für den kleinkalibrigen Gefäßersatz, die in ihrer Langzeitleistung dem autologen Blutgefäß gleichwertig sind, stehen derzeit nicht zur Verfügung. Synthetische Implantate weisen unzureichende biomechanische Eigenschaften auf und begünstigen die Entstehung von Thromben.

Biologisch abbaubare Gefäßprothesen, die nach der Implantation *in situ* durch den Wirt mit gefäßspezifischen Wirtszellen vitalisiert werden, sind ein neuer viel versprechender Therapieansatz. Das Ziel dieser Anwendung ist nach dem vollständigen Abbau des synthetischen Konstruktes letztendlich die Formation eines neuen, funktionellen Blutgefäßes. Wesentlich für den Anwendungserfolg ist aber eine Abstimmung von biologischer Aktivität des Empfängers und den Eigenschaften der Prothese, damit Abbau und Neuersatz durch körpereigenes Gewebe synchronisiert verlaufen.

Kleinlumige Gefäßprothesen, die aus thermoplastischen Polyether- und Polycarbonat-Urethanen mittels Elektrospinnen hergestellt wurden, lieferten bei Tests in präklinischen Studien sehr gute Ergebnisse; siehe etwa Baudis S., Ligon S. C., Seidler K., Weigel G., Grasl C., Bergmeister H., Schima H., Liska R., "Hard-block degradable thermoplastic urethane-elastomers for electrospun vascular prostheses", J. Polym. Sci. A1 50, 1272-1280 (2012); Seidler K., Ehrmann K., Steinbauer P., Rohatschek A., Andriotis O. G., Dworak C., Koch T., Bergmeister H., Grasl C., Schima H., Thurner P.J., Liska R., Baudis S., "A structural reconsideration: Limearalphatic or alicyclic hard segments for biodegradable thermoplastic polyurethanes?", J. Polym. Sci. A1 56, 2214-2224 (2018); sowie Ehrmann K., Potzmann P., Dworak C., Bergmeister H., Eilenberg M., Grasl C., Koch T., Schima H., Liska R., Baudis S., "Hard Block Degradable Polycarbonate Urethanes: Promising Biomaterials for Electrospun Vascular Prostheses", Biomacromolecules 21(2), 376-387 (2020)). In diesen Dokumenten werden verschiedene biologisch abbaubare thermoplastische Polyurethane ("thermoplastic polyurethanes", TPUs), genauer gesagt Polyether- und Polycarbonat-Urethane, offenbart, die durch Polyaddition aus Diisocyanaten, Makrodiolen und "chain extenders", also Kettenverlängerern, hergestellt werden. Darin dienen die von den Kettenverlängerern und von den Diisocyanaten stammenden Reste als Hartsegmente und jene der Makrodiole als Weichsegmente. Die im Stand der Technik offenbarten Materialien verfügen dabei durchwegs über gute thermomechanische Eigenschaften, sind bei ihrer Verwendung zur Herstellung von Gefäßprothesen durch Elektrospinnen gut handhabbar und haben zudem bei Verwendung als Implantate in Tierversuchen gute biologische Abbaubarkeit gezeigt. Für eine erfolgreiche klinische Anwendung wären jedoch Implantate mit weiter verbesserten biomechanischen Eigenschaften wünschenswert, um eine sichere Langzeitanwendung zu gewährleisten.

Zu Polymeren, die oftmals über recht gute mechanische Eigenschaften verfügen, zählen auch Polyurethan-Polyharnstoff-Thermoplaste, die üblicherweise mittels Polyaddition von Diolen und Diaminen mit Diisocyanaten hergestellt werden und hierin in der Folge als thermoplastische Poly(urethan-harnstoff)-Addukte oder kurz TPUUs ("thermoplastic polyurethane-ureas") bezeichnet werden. Die Struktur solcher Addukte kann allgemein durch die nachstehende Formel (1) dargestellt werden:

$$-[(I\text{-}A)_a\text{-}(I\text{-}B)_b]_n- \qquad (1)$$

worin I, A und B jeweils für zweiwertige Reste stehen, die von einem Diisocyanat (I), einem Diol (A) bzw. einem Diamin (B) abgeleitet und jeweils über eine Urethan- oder eine Harnstoffgruppierung miteinander verbunden sind, a und b für die Anzahl der Wiederholungen der Urethan- bzw. Harnstoff-Einheiten stehen und n für die Anzahl an die beiden umfassenden Blöcken steht. Eine beispielhafte detailliertere Darstellung einer Struktur mit jeweils alternierenden Diol-Einheiten A und Diamin-Einheiten B, d. h. mit a = b = 1 ist die nachstehende Formel (2):

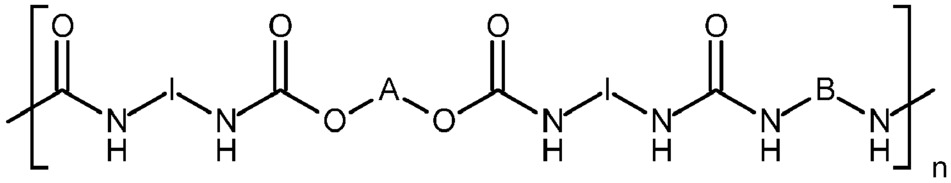

(2)

Darin wechseln sich zudem je zwei von der Addition eines Diols HO-A-OH an zwei Diisocyanat-Moleküle stammende Urethan-Gruppierungen -NH-CO-O- bzw. -O-CO-NH- mit je zwei von der Addition eines Diamins $H_2N$-B-$NH_2$ an zwei Diisocyanate stammenden Harnstoff-Gruppierungen -NH-CO-NH- ab. Stehen a und b jeweils für Zahlen > 1, so wechseln sich innerhalb eines Blocks Polyurethan- mit Polyharnstoff-Segmenten ab.

Etwas komplexer ist die Darstellung bei Verwendung von sekundären Diaminen zur Ausbildung der Harnstoff-Einheiten, da in diesen Fällen neben dem Rest B auch die beiden weiteren, an die Stickstoffatome gebundenen Reste zu erfassen sind. Beispielsweise würde ein N,N'-Dimethyl-Derivat des obigen Diamins eine Struktur gemäß nachstehender Formel (3) ergeben:

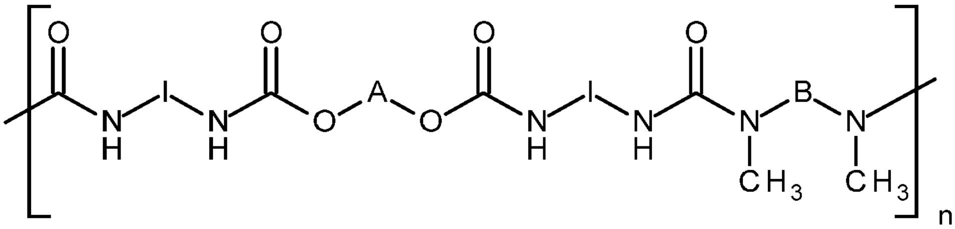

(3)

In vereinfachter Schreibweise könnte die allgemeine Struktur angegeben werden, indem der durch Entfernung der beiden N-gebundenen Wasserstoffatome des Diamins erhaltene zweiwertige Rest -$NCH_3$-B-$NCH_3$- als Rest D bezeichnet wird, wie in Formel (4) dargestellt:

$$-[(I\text{-}A)_a\text{-}(I\text{-}D)_b]_n- \qquad (4)$$

Eine Kombination beider Darstellungsarten ergibt in diesem Fall eine Struktur der folgenden Formel (5):

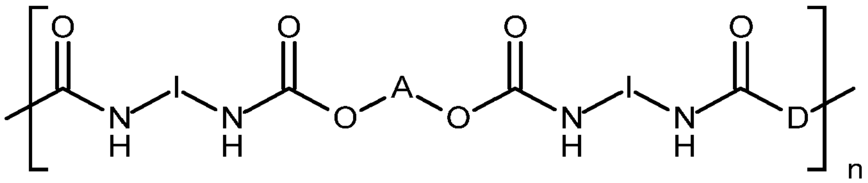

(5)

worin die beiden Stickstoffatome des Rests D jeweils eine N-methylierte HarnstoffGruppierung vervollständigen.

Die mechanischen Eigenschaften von TPUUs lassen sich durch geeignete Auswahl der Monomere in relativ weiten Bereichen steuern, z. B. durch Vorsehen eines geeigneten Verhältnisses zwischen Hart- und Weichsegmenten innerhalb der Polymerketten. So wird beispielsweise in der EP 452.775 A2 die Herstellung von TPUUs mit erhöhter Wärmebeständigkeit unter Verwendung von 4,4'-Diisocyanatodicyclohexylmethan und gegebenenfalls methyliertem Piperazin zur Herstellung von Polyharnstoff-Hartsegmenten offenbart, die mit aus Makrodiolen hergestellten Polyurethan-Weichsegmenten kombiniert werden.

Weiters ist bereits seit Jahrzehnten bekannt, dass in sterisch gehinderten Harnstoffmolekülen die Stabilität der Bindung zwischen mit zumindest einem voluminösen Rest substituierten Stickstoffatomen und der Carbonylgruppe instabil ist. So wurde bereits 1974 die Dissoziation von Harnstoffmolekülen mit verschiedenen sperrigen Substituenten

beschrieben, darunter diverse Kombinationen aus Isopropyl-, sec-Butyl, tert-Butyl-, 3-Pentyl-, Cyclohexyl- und Isooctyl-Substituenten an demselben Stickstoffatom sowie eine zyklische Variante unter Verwendung von Tetramethylpiperidin (Stowell, J. C., Padegimas, S. J., "Urea dissociation. Measure of steric hindrance in secondary amines", J. Org. Chem. 39(16), 2448-2449 (1974)).

Die dabei auftretende Gleichgewichtsreaktion kann am Beispiel einer doppelten Substitution mit tert-Butyl wie im folgenden Schema A dargestellt werden:

Schema A

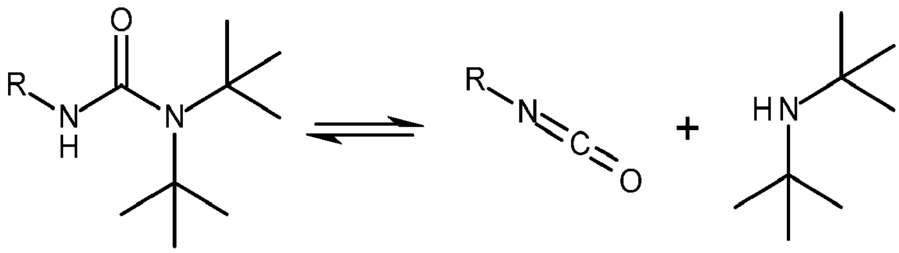

Dieses Schema veranschaulicht, dass die instabile Bindung zwischen der Carbonylgruppe und dem sterisch gehinderten Stickstoffatom des sekundären Amins unter bestimmten Bedingungen unter Ausbildung eines Isocyanats und des freien sekundären Amins reversibel gespalten wird, d. h. es bildet sich auch wieder der ursprüngliche Harnstoff zurück, wobei die Lage des Gleichgewichts maßgeblich von der Auswahl der beiden Substituenten am sterisch gehinderten Stickstoffatom abhängt.

Sterisch gehinderte Harnstoffgruppen wurden in der Folge unter anderem zur Maskierung von Isocyanaten verwendet; siehe beispielsweise Hutchby, M., Houlden, C. E., Ford, J. G., Tyler, S. N. G., Gagne, M. R., Lloyd-Jones, G. C., Booker-Milburn, K. I., "Hindered ureas as masked isocyanates: facile carbamoylation of nucleophiles under neutral conditions", Angew. Chem. Int. Ed. 48(46), 8721-8724 (2009)). Die erstmalige Nutzung dieser Reaktion in Makromolekülen für die Entwicklung "dynamischer" Materialien wird in Ying, H., Zhang, Y., Cheng, J., "Dynamic urea bond for the design of reversible and self-healing polymers", Nat. Commun. 5, 3218 (2014), beschrieben. Dabei wurden für molekulare Kinetikstudien 2,2,6,6-Tetramethylpiperidinylcarbonsäureamid und 1-tert-Butyl-1-isopropyl-, 1-tert-Butyl-1-ethyl-, 1,1-Diisopropyl- und 1,1-Diethylharnstoff eingesetzt. Weiters wurden ein einfaches Polyharnstoff-Molekül aus 1,3-Bis(isocyanatomethyl)cyclohexan und N,N'-Di-tert-butylethylendiamin sowie vernetzte Polyurethan-Harnstoff-Polymere aus Triethanolamin, Hexamethylendiisocyanat, Tetraethylenglykol und vier verschiedenen, sterisch gehinderten Diaminen hergestellt und untersucht, wobei sog. "selbstheilende Effekte" des vernetzten Polymermaterials festgestellt werden konnten, die jedoch stets mit einer Abnahme der Zugfestigkeit durch die Selbstheilung einhergingen.

In der Folge wurden zahlreiche weitere Experimente mit entsprechenden "selbstheilenden" Polymeren durchgeführt. So verwendeten etwa Zhang et al. verschiedene Ethanolamine, nämlich 2-tert-Butylaminoethanol, 2-Isopropylaminoethanol und N-Butylaminoethanol, zur Herstellung von sog. "recycelbaren" Poly(urethan-harnstoff)-Duroplasten (Zhang, Y., Ying, H., Hart, K. R., Wu, Y., Hsu, A. J., Coppola, A. M., Kim, T. A., Yang, K., Sottos, N. R., White, S. R., Cheng, J., "Malleable and recyclable poly-(urea-urethane) thermosets bearing hindered urea bonds", Adv. Mater. 28(35), 7646-7651 (2016)). Die recycelten Polymermaterialien erreichten dabei jedoch bestenfalls die ursprünglichen Zugfestigkeitswerte. Weiters beschrieben Zhang et al. erneut die Verwendung von Ethanolaminen als Kettenverlängerer für dynamische Poly(alkylharn-stoff-urethan)-Netzwerke mit verbessertem Spannungsabbau (Zhang, L., Rowan, S. J., "Effect of sterics and degree of cross-linking on the mechanical properties of dynamic poly(alkylurea-urethane) networks", Macromolecules 50(13), 5051-5060 (2017)). Bruce und Lewis untersuchten Glasübergangstemperaturen von selbstheilenden Poly-(urethan-harnstoffen) zur Herstellung von weniger weichen Materialien, die denselben Selbstheilungsmechanismus aufweisen (Bruce, A. C., Lewis, C. L., "Influence of glass transition temperature on mechanical and self-healing behavior of polymers bearing hindered urea bonds", SPE ANTEC, Anaheim, 2017; Anaheim, 2017), und zwei weitere Artikel betreffen die Verwendung von sterisch gehinderten Diaminen (v. a. N,N'-Di-tert-butylethylendiamin) als Kettenverlängerer zur Herstellung von reprozessierbaren Poly(urethan-harnstoff)-Duroplasten mit Formgedächtnis (Fang, Z., Zheng, N., Zhao, Q., Xie, T., "Healable, reconfigurable, reprocessable thermoset shape memory polymer with highly tunable topological rearrangement kinetics", ACS Appl. Mater. Interfaces 9(27), 22077-22082 (2017); Wang, Y., Pan, Y., Zheng, Z., Ding, X., "Reconfigurable and reprocessable thermoset shape memory polymer with synergetic triple dynamic covalent bonds", Macromol. Rapid Commun. 39(10), 1800128 (2018)).

Auch in der Patentliteratur finden sich entsprechende Offenbarungen zu solchen "selbstheilenden" Polymeren, vor allem auch von den obigen Autoren als Erfinder; siehe beispielsweise WO 2014/144539 A2, WO 2016/069582 A1, WO 2016/126103 A1 und WO 2016/126756 A1.

Das bei der in Schema A dargestellten Reaktion entstehende Isocyanat erfährt jedoch in wässrigem Milieu eine Hydrolyse zur entsprechenden Carbaminsäure, die anschließend zu spontaner Decarboxylierung neigt, wie dies nachstehend in Schema B dargestellt ist:

Schema B

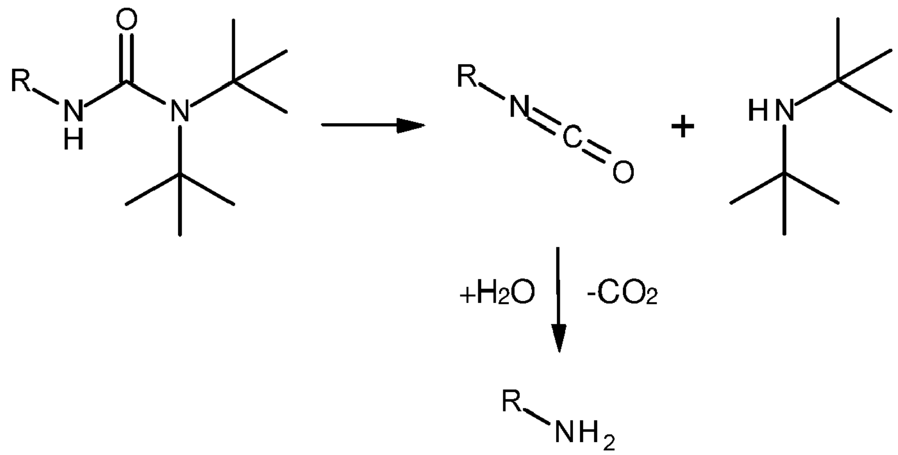


Dabei entstehen die beiden freien Amine, die ursprünglich das Harnstoff-Molekül gebildet hatten. Eine solche In-situ-Entstehung von Isocyanaten aus sterisch gehinderten Harnstoffmolekülen und anschließende Hydrolyse der Isocyanate in wässrigem Medium ermöglicht unter anderem die Hydrolyse von Polyharnstoffen.

Für Makromoleküle wurde diese Reaktionsabfolge erstmalig von Ying et al. offenbart (Ying, H., Cheng, J., "Hydrolyzable polyureas bearing hindered urea bonds", J. Am. Chem. Soc. 136(49), 16974-16977 (2014)), wobei Polyharnstoffe sowie vernetzte Poly-(urethan-harnstoff)-Organogele in DMF gelöst und anschließend mit Wasser hydrolysiert wurden, und in weiterführenden Arbeiten wurde die Biokompatibilität von Hydrogelen, die sterisch gehinderte Urethangruppen zur Hydrolyse enthielten, belegt (siehe Ying, H., Yen, J., Wang, R., Lai, Y., Hsu, J.-L.-A., Hu, Y., Cheng, J., "Degradable and biocompatible hydrogels bearing a hindered urea bond", Biomater. Sci. 5(12), 2398-2402 (2017)). Später untersuchten Cai et al. die Hydrolyse eines in THF mit 5 Vol.-% Wasser gelösten Polyharnstoffs und wiesen dabei die pH-Unabhängigkeit der Hydrolysereaktion nach (Cai, K., Ying, H., Cheng, J., "Dynamic Ureas with fast and pHindependent hydrolytic kinetics", Chem. Eur. J. 24(29), 7345-7348 (2018); sowie WO 2017/155958 A1), und kürzlich offenbarten Chen et al. die Verwendung von Polyharnstoffen mit sterisch gehinderten Harnstoffgruppen zur Wirkstofffreisetzung (Chen, M., Feng, X., Xu, W., Wang, Y., Yang, Y., Jiang, Z., Ding, J., "PEGylated polyurea bearing hindered urea bond for drug delivery", Molecules 24(8), 1538 (2019)), wobei mehrere PEGylierte Polyharnstoffe in Lösung in DMSO zur Ausbildung von Micellen rund um einen Anti-Tumor-Wirkstoff (Paclitaxel) eingesetzt wurden, eine Lösung dieser Micellen in PBS angefertigt und in Tumoren von Versuchstieren injiziert wurde, wo durch Hydrolyse der Polyharnstoff-Moleküle der Wirkstoff freigesetzt wurde.

Bei all diesen publizierten Versuchen zur "Selbstheilung" bzw. "Nachbehandlung" von Polyharnstoffen und Poly(urethan-harnstoffen), von denen die überwiegende Mehrzahl nur die reversible Reaktion gemäß Schema A und nur wenige auch die anschließende Hydrolysereaktion zum Gegenstand haben, konnten zwar in seltenen Fällen Teilverbesserungen einzelner mechanischer Eigenschaften erzielt werden, wie z.B. die oben erwähnten Poly(urethanharnstoffe) (PUUs) mit verbessertem Spannungsabbau gemäß Zhang et al., zumeist wurden jedoch modifizierte Polymere erhalten, die schlechtere Eigenschaften als davor oder bestenfalls gleich gute aufwiesen. Zudem wurden noch niemals speziell thermoplastische Polyharnstoffe, Polyurethane oder Poly(urethan-harnstoff)-Polyaddukte (TPUUs) unter diesem Aspekt untersucht, und folglich wurde auch in keinem einzigen Fall über modifizierte Polymere mit Eigenschaften berichtet, die sich für die eingangs erwähnten thermomechanischen Verarbeitungsprozesse besser eignen würden als das jeweilige Ausgangsmaterial.

In der noch unveröffentlichten europäischen Patentanmeldung EP 20183957 einer der Anmelderinnen werden neuartige TPUU-Polyaddukte mit sterisch gehinderten Harnstoffgruppen der nachstehenden Formel (I) beschrieben:

$$-[I-M-(I-C_1)_a-(I-M)_b-(I-C_2)_c]_n- \quad (I)$$

worin I, M, $C_1$ und $C_2$ jeweils für zweiwertige Reste stehen, die jeweils über eine Urethan- oder eine Harnstoffgruppierung miteinander verbunden sind, wovon

I jeweils unabhängig für einen zweiwertigen, von einem Diisocyanat abgeleiteten, gesättigten oder ungesättigten, aliphatischen, alicyclischen oder aromatischen Rest mit 1 bis 20 Kohlenstoffatomen steht;

M jeweils unabhängig für einen zweiwertigen, von einem Makrodiol abgeleiteten Rest eines aliphatischen Polyethers, Polyesters oder Polycarbonats mit einem zahlenmittleren Molekulargewicht $M_n \geq 500$ steht;

$C_1$ jeweils unabhängig für einen zweiwertigen, von einem Diamin oder Aminoalkohol mit jeweils zumindest einer sterisch gehinderten sekundären Aminogruppe durch Entfernung je eines N-gebundenen Wasserstoffatoms des Diamins oder eines N-gebundenen und des O-gebundenen Wasserstoffatoms des Aminoalkohols abgeleiteten, gesättigten oder ungesättigten, aliphatischen oder alicyclischen Rest mit 1 bis 30 Kohlenstoffatomen steht;

$C_2$ jeweils unabhängig für einen zweiwertigen, von einem Diol, Diamin oder Aminoalkohol ohne sterisch gehinderte Aminogruppen abgeleiteten, gesättigten oder ungesättigten, aliphatischen, alicyclischen oder aromatischen Rest mit 1 bis 20 Kohlenstoffatomen steht;

wobei in den Resten von I, $C_1$ und $C_2$ bei Vorliegen von mehr als vier Kohlenstoffatomen gegebenenfalls zumindest eines davon durch ein aus Sauerstoff und Stickstoff ausgewähltes Heteroatom ersetzt ist;

wobei zumindest einer der Reste I, M, $C_1$ und $C_2$ eine oder mehrere Estergruppierungen umfasst; und

a, b und c jeweils unabhängig für eine ganze Zahl von 0 bis 10 stehen und n eine Zahl $\geq 3$ ist, die für die Anzahl der Blöcke des Polyaddukts steht;

mit der Maßgabe, dass jeweils innerhalb desselben Blocks $a + c \geq 1$ ist und in allen Blöcken zusammen zumindest ein $a \geq 1$ ist und zumindest ein $c \geq 1$ ist.

Für solche thermoplastische Poly(urethan-harnstoff)- (TPUU-) Polyaddukte mit sterisch gehinderten Harnstoffgruppen wurde herausgefunden, dass diese Makromoleküle nicht nur in Lösung oder als Hydrogel, wie dies aus dem Stand der Technik bekannt war, sondern auch im festen Zustand bei Kontakt mit Wasser oder in wässriger Umgebung die in Schema B dargestellte Reaktion zeigen - und das sogar nach Verarbeitung des Materials zu festen Erzeugnissen, wie z.B. durch Lösungsgießen, Folienziehen oder dergleichen.

Dabei wird das durch die Öffnung der labilen Harnstoffbindung gebildete Isocyanat bei Verwendung dieser TPUUs in festem Zustand nicht wie in Schema B vollständig zum freien Amin hydrolysiert, sondern ein Teil des Isocyanats reagiert mit einem Teil des durch die Hydrolyse entstandenen freien Amins unter Ausbildung einer neuen, nicht sterisch gehinderten Harnstoffgruppierung, woraus stabile Polymerketten mit verbesserten thermomechanischen Eigenschaften resultieren. Das Auftreten dieser - als "Rekombinationsreaktion" bezeichneten - Reaktion eines decarboxylierten Isocyanats mit einem noch nicht decarboxylierten wird darin auf die verringerte Reaktionsgeschwindigkeit der Hydrolysereaktion der Polymerketten in der festen Phase zurückgeführt. In Schema C ist eine solche Reaktionsabfolge - analog zu Schema B - für eine endständige, sterisch gehinderte Harnstoffgruppe dargestellt:

Schema C

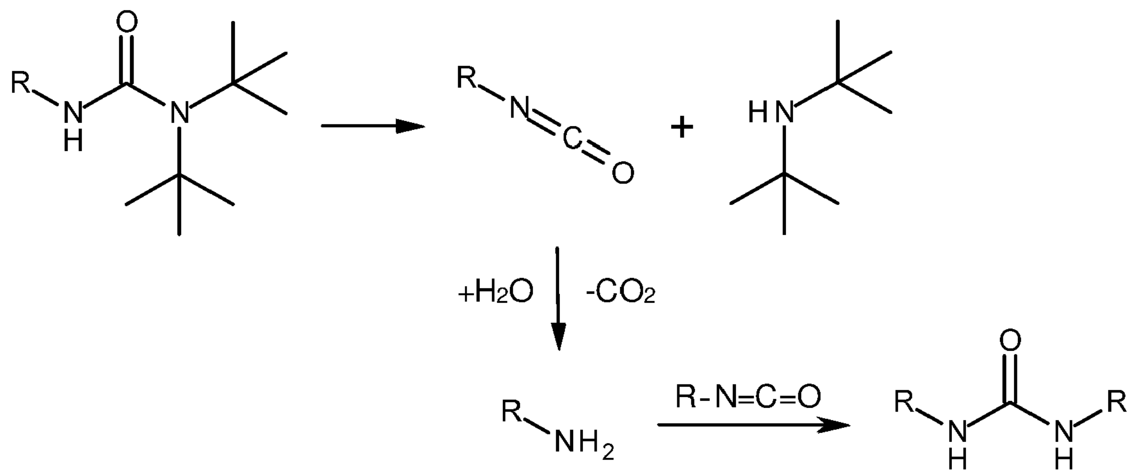


Und in Schema D für zwei Harnstoffgruppierungen inmitten einer TPUU-Polymerkette, die von demselben, beidseitig sterisch gehinderten sekundären Diamin, im vorliegenden Fall von N,N'-Di-tert-butylpropylendiamin, herrühren:

Schema D

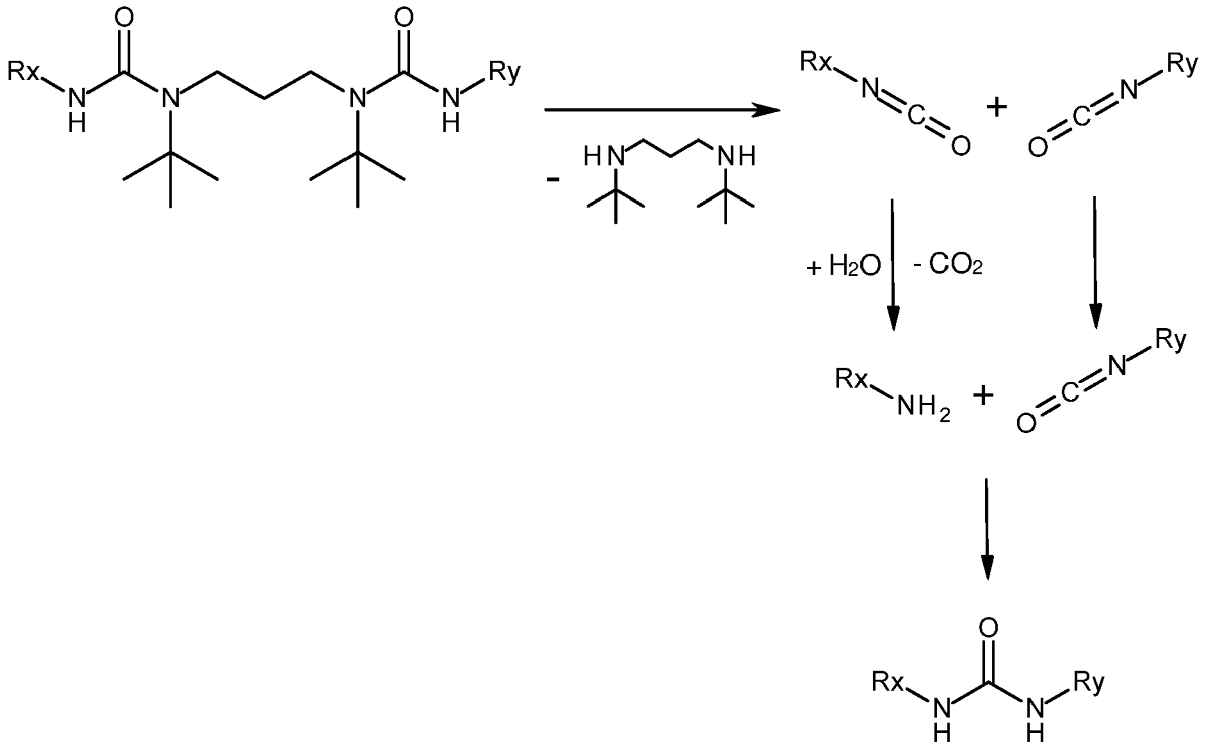


Auf diese Weise bildet sich bei Kontakt des TPUUs mit Wasser ein neues TPUU-Polymer, das formal aus der Eliminierung eines beidseitig sterisch gehinderten Diamins und einer Carbonylgruppe aus der Hauptkette des Polymers resultiert.

Die Reaktion von TPUUs, die Reste von nur einseitig sterisch gehinderte Diaminen oder Aminoalkoholen enthalten, ist im umseitigen Schema E dargestellt, worin X für O (von einem Aminoalkohol stammend) oder NH (von einem Diamin stammend) steht:

Schema E

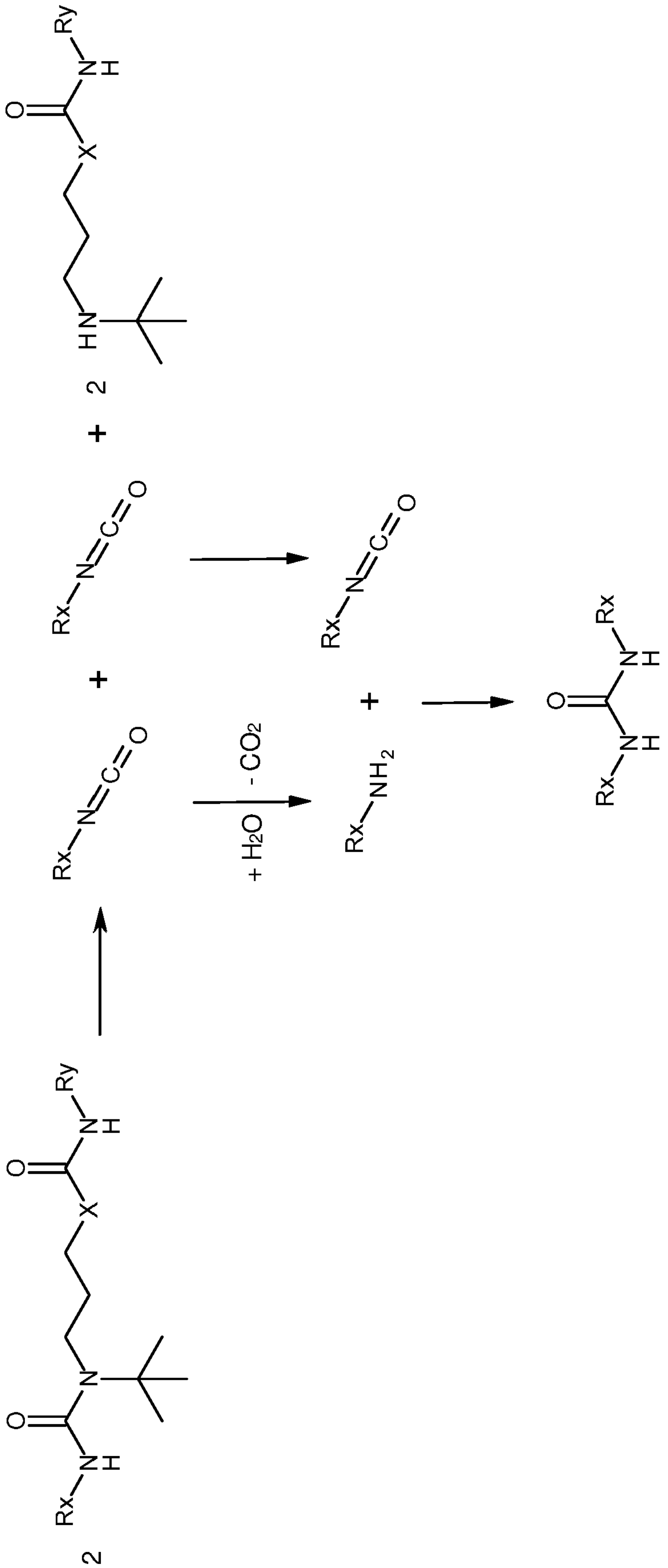


Durch Bindungsöffnung jeweils einer sterisch gehinderten Harnstoffgruppierung zweier TPUU-Polymerketten

entstehen zwei polymere Isocyanate Rx-N=C=O, von denen eines in Gegenwart von Wasser die Hydrolyse und Decarboxylierung zum freien Amin Rx-$NH_2$ erfährt, das jedoch in der Folge an das zweite, noch nicht decarboxylierte Isocyanat addiert und dadurch unter Verdoppelung der Kettenlänge des Rests Rx-NHein neues, stabiles TPUU-Polymer bildet. Weiters entstehen zwei, gegenüber dem Ausgangsmolekül um die Länge von Rx verkürzte neue TPUU-Polymere mit endständigen, sterisch gehinderten sekundären Aminogruppen, die zwar gegebenenfalls mit einem der Isocyanate die zuvor bekannte, "selbstheilende" Rückreaktion zum instabilen Ausgangspolymer eingehen, allerdings nicht in der Lage sind, mit Isocyanaten stabile Harnstoffbindungen auszubilden.

Auf diese Weise bilden sich aus den TPUUs der Formel (I) in festem Zustand in wässrigem Milieu jeweils neue Polymere, die je nach der Lage der sich öffnenden instabilen Bindung(en) innerhalb der Hauptketten größere Kettenlängen (Schema C), im Wesentlichen dieselben Kettenlängen (Schema D) oder sowohl größere als auch kürzere Kettenlängen (Schema E) als die Ausgangsmoleküle aufweisen können, die jedoch in allen Fällen nach der Reaktion mit Wasser ausschließlich stabile, nicht sterisch gehinderte Harnstoffbindungen umfassen.

Aus den zuvor erwähnten wässrigen Lösungen von Polyharnstoffen entstehen hingegen ausschließlich kürzere Moleküle, da die Hydrolyse und anschließende Decarboxylierung der durch die Öffnung instabiler, sterisch gehinderter Harnstoffbindungen gebildeten Isocyanate in Lösung viel rascher abläuft, so dass diese nicht ausreichend lange stabil sind, um eine Reaktion mit einem freien Amin einzugehen. Die in Schema D dargestellte Reaktion von TPUUs in festem Zustand bei Kontakt mit Wasser, die zu einem beidseitig sterisch gehinderten, freien sekundären Diamin sowie einer "rekombinierten" und nur um die Länge des Diamins und einer Carbonylgruppe verkürzten Polyharnstoffkette führt, würde in Lösung neben dem freigesetzten sterisch gehinderten Diamin lediglich zwei polymere freie Amine ergeben, zwischen denen freilich keine Reaktion erfolgen kann, wie im nachfolgenden Schema F dargestellt:

Schema F

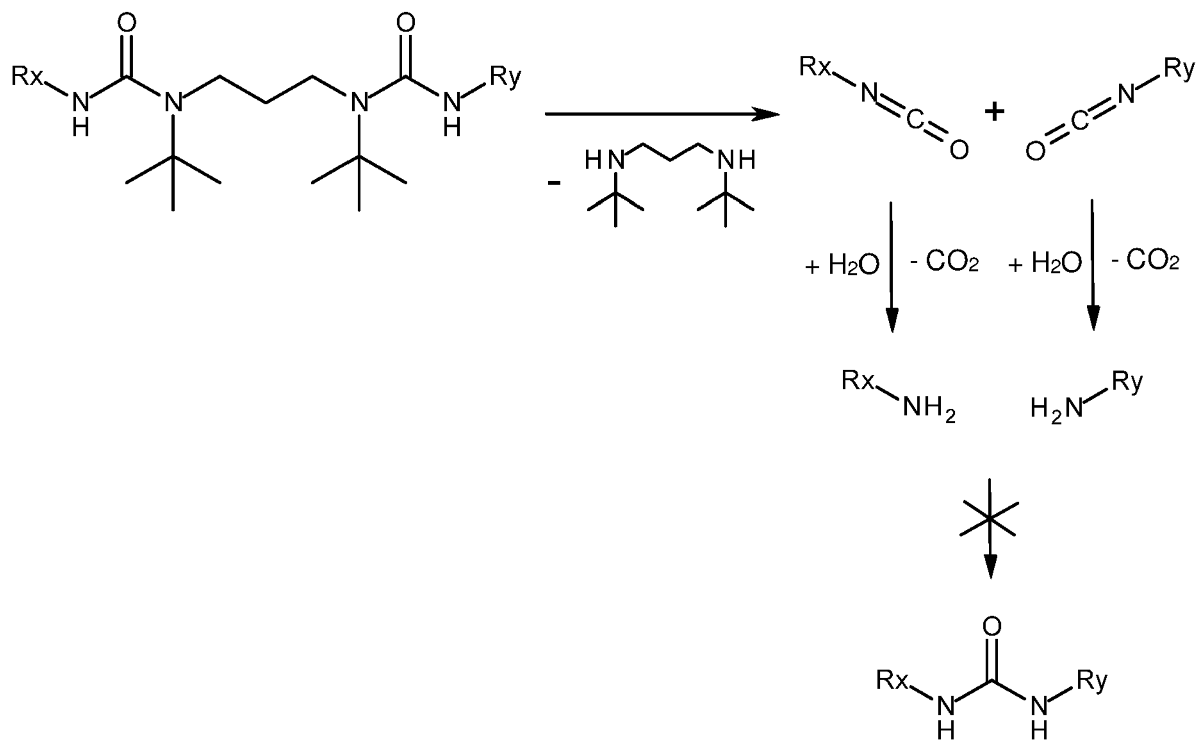


Dabei entstehen zwei neue Polymere, deren Molekulargewichte, je nach Position des vom sterisch gehinderten sekundären Diamin stammenden Rests innerhalb der ursprünglichen Polymerkette, geringer sind als jenes des Ausgangspolymers und das mitunter deutlich, bis hin zu einer etwa 50%igen Reduktion. Auch aus diesem Grund wurden in nahezu allen einschlägigen Dokumenten des älteren Standes der Technik, die über das Verhalten von "selbstheilenden" Polyharnstoffen in wässriger Lösung berichten, bestenfalls gleichbleibende, zumeist aber verschlechterte thermomechanische Eigenschaften der entstandenen kürzeren Polymere festgestellt.

Darüber hinaus weisen die gemäß obigem Schema D aus TPUUs der Formel (I) in festem Zustand bei Kontakt mit Wasser (oder einem wässrigen Milieu) erhaltenen neuen Polymere Rx-NH-CO-NH-Ry jedoch - unter der Annahme, dass in der Polymerkette nur ein sterisch gehindertes Diamin enthalten war - praktisch dasselbe Molekulargewicht wie vor der Behandlung mit Wasser auf und enthalten auch eine neue Harnstoffgruppierung, deren beiden Wasserstoffatome

nicht durch voluminöse Reste abgeschirmt sind und daher zur Ausbildung von Wasserstoffbrücken zu Harnstoff- oder Urethangruppierungen einer weiteren, benachbarten Polymerkette in der Lage sind. Diese Wasserstoffbrücken werden als Hauptgrund für die Verbesserung der thermomechanischen Eigenschaften angesehen, da dieser Effekt selbst dann auftritt, wenn eine Vielzahl von sterisch gehinderten Harnstoffgruppierungen innerhalb der Ketten der TPUUs der Formel (I) enthalten ist, so dass durch die "Rekombinationsreaktionen" durchwegs neue Ketten mit (mitunter sogar deutlich) niedrigerem Molekulargewicht entstehen.

TPUUs der Formel (I) weisen bei Verarbeitung zu festen Produkten bereits nach wenigen Stunden der Lagerung der Produkte in Wasser bessere Dehnbarkeits- und Zugfestigkeitswerte sowie höhere Schmelzpunkte als unmittelbar nach der Verarbeitung auf. Gleichzeitig war die Löslichkeit vor der Wasserbehandlung höher, woraus eine einfachere Verarbeitbarkeit der Ausgangspolymere resultiert.

Aufgrund der Gegenwart von Estergruppierungen in den Ketten dieser TPUUs sind sowohl die Ausgangspolymere als auch deren durch "Rekombination" gebildeten Reaktionsprodukte in einem geeigneten wässrigen Milieu, wie z.B. unter physiologischen Bedingungen, durchwegs spaltbar, was den Vorteil der biologischen Abbaubarkeit mit sich bringt.

Die in Formel (I) enthaltenen Komponenten stehen als Abkürzungen, wie in der Polyurethan- und Polyharnstoff-Chemie üblich, für Isocyanat "I", Makrodiol "M", sowie zwei unterschiedliche Arten von Amino- bzw. OH-Gruppen enthaltenden "Chain Extendern", also "Kettenverlängerern", "$C_1$" und "$C_2$", die als Hartsegmente zur Verknüpfung von Isocyanat- und Makrodiol-Bausteinen über entsprechende Urethan- und/oder Harnstoffbindungen dienen. Von diesen beiden Kettenverlängerern dient, wie oben definiert, $C_1$ als ein- oder beidseitig sterisch gehindertes Diamin oder als Aminoalkohol mit sterisch gehinderter sekundärer Aminogruppe zur Ausbildung der instabilen und in wässriger Umgebung spaltbaren Harnstoffbindungen. Und $C_2$ dient als weiteres Hartsegment einerseits zur zusätzlichen Verknüpfung von Makrodiol-Bausteinen und somit zur Steuerung der Kettenlänge zwischen den von $C_1$ gebildeten sterisch gehinderten Harnstoffgruppierungen, mitunter aber zusätzlich auch zur Förderung der biologischen Abbaubarkeit, wenn als Monomerbausteine zur Einführung von $C_2$ in die TPUU-Kette Diamine, Diole bzw. Aminoalkohole ausgewählt werden, die eine unter physiologischen Bedingungen spaltbare Estergruppierung enthalten. Dies ist insbesondere vorteilhaft, wenn als Makrodiol ein Polyether ohne spaltbare Carboxylat- oder Carbonat-Estergruppierungen gewählt wird.

Vor diesem Hintergrund war das Ziel der vorliegenden Erfindung die Entwicklung eines neuen Verfahrens zur Herstellung von Gefäßprothesen mit gegenüber jenen der zuvor genannten TPUs verbesserten biomechanischen Eigenschaften.

OFFENBARUNG DER ERFINDUNG

Dieses Ziel erreicht die vorliegende Erfindung im ersten Aspekt durch Bereitstellung der Verwendung eines thermoplastischen Poly(urethan-harnstoff)-Polyaddukts mit sterisch gehinderten Harnstoffgruppen der Formel (I) in einem Elektrospinn-Verfahren zur Herstellung von Gefäßprothesen:

$$-[I\text{-}M\text{-}(I\text{-}C_1)_a\text{-}(I\text{-}M)_b\text{-}(I\text{-}C_2)_c]_n- \qquad \text{(I)}$$

worin, wie oben beschrieben,

I, M, $C_1$ und $C_2$ jeweils für zweiwertige Reste stehen, die jeweils über eine Urethan- oder eine Harnstoffgruppierung miteinander verbunden sind, wovon

I jeweils unabhängig für einen zweiwertigen, von einem Diisocyanat abgeleiteten, gesättigten oder ungesättigten, aliphatischen, alicyclischen oder aromatischen Rest mit 1 bis 20 Kohlenstoffatomen steht;

M jeweils unabhängig für einen zweiwertigen, von einem Makrodiol abgeleiteten Rest eines aliphatischen Polyethers, Polyesters oder Polycarbonats mit einem zahlenmittleren Molekulargewicht $M_n \geq 500$ steht;

$C_1$ jeweils unabhängig für einen zweiwertigen, von einem Diamin oder Aminoalkohol mit jeweils zumindest einer sterisch gehinderten sekundären Aminogruppe durch Entfernung je eines N-gebundenen Wasserstoffatoms des Diamins oder eines N-gebundenen und des O-gebundenen Wasserstoffatoms des Aminoalkohols abgeleiteten, gesättigten oder ungesättigten, aliphatischen oder alicyclischen Rest mit 1 bis 30 Kohlenstoffatomen steht;

$C_2$ jeweils unabhängig für einen zweiwertigen, von einem Diol, Diamin oder Aminoalkohol ohne sterisch gehinderte sekundäre Aminogruppen abgeleiteten, gesättigten oder ungesättigten, aliphatischen, alicyclischen oder aromatischen Rest mit 1 bis 20 Kohlenstoffatomen steht;

wobei in den Resten von I, $C_1$ und $C_2$ bei Vorliegen von mehr als vier Kohlenstoffatomen gegebenenfalls zumindest eines davon durch ein aus Sauerstoff und Stickstoff ausgewähltes Heteroatom ersetzt ist;

wobei zumindest einer der Reste I, M, $C_1$ und $C_2$ eine oder mehrere Estergruppierungen umfasst; und

a, b und c jeweils unabhängig für eine ganze Zahl von 0 bis 10 stehen und n eine Zahl $\geq 3$ ist, die für die Anzahl der Blöcke des Polyaddukts steht;

mit der Maßgabe, dass jeweils innerhalb desselben Blocks $a + c \geq 1$ ist und in allen Blöcken zusammen zumindest ein $a \geq 1$ ist und zumindest ein $c \geq 1$ ist.

Diese thermoplastischen Poly(urethan-harnstoff)-Polyaddukte der Formel (I) mit sterisch gehinderten Harnstoffgruppen stellen eine neue Generation von Biomaterialien dar, da sie aufgrund der oben erwähnten, bei Kontakt mit Wasser oder in wässriger Umgebung, z.B. bei Kontakt mit Blut, erfolgenden Ausbildung neuer Polymerketten gemäß den Schemata C bis E über "selbstverstärkende" Eigenschaften verfügen. Unter Verwendung eines solchen Materials ist es gemäß vorliegender Erfindung möglich, durch Elektrospinnen Gefäßprothesen herzustellen, deren biomechanischen Eigenschaften jenen der Implantate aus den eingangs zitierten, bekannten Materialien überlegen sind.

Analog zu EP 20183957 ist auch in bevorzugten Ausführungsformen der vorliegenden Erfindung in dem TPUU der Formel (I) zumindest ein Teil der Estergruppierungen unter physiologischen Bedingungen spaltbar, sind die Reste I, M, $C_1$ und $C_2$ sowie etwaige Spaltprodukte davon biokompatibel und physiologisch unbedenklich und wird durch das Elektrospinn-Verfahren eine temporäre Gefäßprothese hergestellt, die im Körper des Empfängers allmählich biologisch abgebaut und durch ein körpereigenes Gefäß ersetzt wird.

In weiteren bevorzugten Ausführungsformen der erfindungsgemäßen Verwendung gilt für die TPUUs der Formel (I), dass

a und c jeweils unabhängig $\leq 5$ oder $\leq 3$ sind; und/oder
a und c jeweils unabhängig $\geq 1$ sind; und/oder
$b \geq 1$ ist; und/oder
$b = c$ oder $b = a$ oder $b + 1 = a + c$ ist; und/oder
$n \geq 5$ oder $n \geq 10$ oder $n \geq 50$ ist.

Dabei bewirken relativ niedrige Werte für a und c einen vergleichsweise hohen Anteil an den von Makrodiolen stammenden Einheiten M, d. h. an Weichsegmenten, in den TPUUs, was für niedrige Schmelzpunkte der Polymere und hohe Flexibilität auch bei relativ niedrigen Temperaturen sowie für eine nicht übermäßig hohe Anzahl an instabilen Harnstoffbindungen im gesamten Polymer sorgt, um bei der Reaktion der Gefäßprothesen im Körper des Patienten keine allzu kurzkettigen Reaktionsprodukte zu erhalten, um die thermomechanischen Eigenschaften der Gefäßprothese nicht zu beeinträchtigen.

Sind sowohl a als auch c jeweils $\geq 1$, sind in jedem Block sowohl zumindest eine von $C_1$ gebildete, sterisch gehinderte Harnstoffgruppe als auch ein weiterer Kettenverlängerer $C_2$, der vorzugsweise eine spaltbare Estergruppierung umfasst, enthalten, was die biologische Abbaubarkeit erhöht.

In TPUUs der Formel (I) mit $b \geq 1$, was vorzugsweise für den Fall, dass a und c jeweils unabhängig $\geq 1$ sind, gilt, sind die beiden Kettenverlängerer-Einheiten $C_1$ und $C_2$ durch zumindest eine Makrodiol-Einheit voneinander getrennt. Dies verbessert die Steuerbarkeit des Abstands zwischen diesen beiden Einheiten und ermöglicht in besonders bevorzugten Ausführungsformen, einen relativ großen Abstand zwischen sterisch gehinderten Harnstoffgruppierungen in $C_1$ und spaltbaren Estergruppierungen in $C_2$ vorzusehen, damit es bei den nach der Implantation der Prothese auftretenden Rekombinationsreaktionen der freien Amine mit nicht decarboxylierten Isocyanatgruppierungen nicht auch zu einer verfrühten Spaltung von Estergruppierungen durch Angriff der freien Amine kommt.

Ausführungsformen, in denen $b = c$ oder $b = a$ oder $b + 1 = a + c$ ist, bieten vor allem Vorteile bei der Herstellung der TPUUs der Formel (I). So kann beispielsweise in den beiden ersten Fällen ein Oligomer oder Präpolymer mit alternierenden, mittels Diisocyanaten verknüpften Kettenverlängerer-Einheiten $C_1$ oder $C_2$ und Makrodiol-Einheiten M durch Vermischen des entsprechenden sterisch gehinderten Diamins oder Aminoalkohols (für $C_1$) oder nicht sterisch gehinderten Diamins oder Aminoalkohols oder Diols (für $C_2$) mit einer äquimolaren Mengen an Makrodiol mit einem geringfügigen molaren Überschuss an Diisocyanat hergestellt werden, bevor das Reaktionsprodukt mit den jeweils gewünschten molaren Mengen des anderen Kettenverlängerers und an Diisocyanat umgesetzt wird. Und im Fall von $b + 1 = a + c$ können einerseits zwei solcher Oligomere oder Präpolymere, die jeweils einen Kettenverlängerer mit Makrodiol-Einheiten alternierend enthalten, auf einfache Weise getrennt voneinander hergestellt und anschließend über Diisocyanat miteinander verbunden werden. Und andererseits können in besonders bevorzugten Ausführungsformen, in denen a, b und c jeweils 1 sind, äquimolare Mengen an die beiden Kettenverlängerer $C_1$ und $C_2$ bereitstellenden Monomerbausteinen mit der doppelten Menge an Makrodiol und der vierfachen Menge an Diisocyanat (oder vorzugsweise einem geringen Überschuss an Diisocyanat) umgesetzt werden, d. h. in einem Verhältnis $C_1 : C_2 : M : I$ von 1 : 1 : 2 : 4 (oder vorzugsweise > 4, z. B. 4,02 oder 4,03), was die Synthese vereinfacht.

Aus dieser Option der Polymerisationsreaktionsführung folgt jedoch für den Fachmann auch, dass die Reihenfolge der Komponenten innerhalb eines Blocks, insbesondere jene der Komponenten $C_1$ und $C_2$, nicht auf die in Formel (I) explizit dargestellte beschränkt ist. Das bedeutet, dass bei Werten für a und c von jeweils > 1, selbst wenn a = c ist, sich die beiden, jeweils einen der Kettenverlängerer enthaltenden Bausteine (I-$C_1$) und (I-$C_2$) innerhalb eines Blocks nicht zwingend abwechseln müssen, sondern auch statistisch verteilt sein können.

Die Anzahl der Blöcke n der TPUUs der Formel (I) - und damit deren zahlenmittleres Molekulargewicht - ist nicht speziell eingeschränkt und kann in Abhängigkeit von den gewünschten thermomechanischen oder sonstigen physikalischen Eigenschaften frei gewählt werden. Wie dem Fachmann wohlbekannt ist, hängt die Kettenlänge von Polyaddukten vor allem von der Stöchiometrie der Monomer- oder Präpolymerbausteine während der Polyadditionsreaktionen ab. Vorzugsweise ist die Anzahl der Blöcke n $\geq$ 5, noch bevorzugter $\geq$ 10 und insbesondere $\geq$ 20, $\geq$ 50 oder $\geq$ 100, was die Verarbeitbarkeit durch Elektrospinnen erhöht.

In weiteren Ausführungsformen der Erfindung sind in den TPUUs der Formel (I):

die Reste I vorzugsweise jeweils unabhängig von einem Diisocyanat aus der aus den folgenden bestehenden Gruppe abgeleitet: 1,6-Hexamethylendiisocyanat, 4,4'-Diisocyanatodicyclohexylmethan, Isophorondiisocyanat, 1,3-Bis(isocyanatomethyl)cyclohexan, Diphenylmethan-4,4'-diisocyanat, L-Lysinethylesterdiisocyanat, obwohl die Diisocyanate nicht speziell eingeschränkt sind, solange die daraus resultierenden Einheiten I jeweils 1 bis 30 Kohlenstoffatome aufweisen; und/oder

die Reste M vorzugsweise jeweils unabhängig von einem Polyether, Polyester oder Polycarbonat aus der aus den folgenden bestehenden Gruppe abgeleitet: Polytetrahydrofuran, Polyethylenglykol, Polypropylenglykol, Polycaprolacton, Polylactid, Polyglykolid, Poly(lactid-co-glykolid), Polyhexamethylencarbonat,

wenngleich auch die Makrodiole, abgesehen von einem zahlenmittleren Molekulargewicht $M_n \geq 500$ der Einheiten M, um für geeignete Molekulargewichte sowie Thermoplastizität der TPUUs der Formel (I) zu sorgen, nicht speziell eingeschränkt sind.

Auch die zur Einführung der zweiwertigen Reste mit jeweils zumindest einer sterisch gehinderten sekundären Aminogruppe eingesetzten Diamine oder Aminoalkohole sind nicht speziell eingeschränkt, solange sie zusätzlich zu den beiden Stickstoffatomen oder dem Stickstoff- und dem Sauerstoffatom ausschließlich gesättigte oder ungesättigte, aliphatische oder alicyclische Reste mit insgesamt 1 bis 30 Kohlenstoffatomen aufweisen. Damit ist hierin die Gesamtheit der Kohlenstoffatome aller Reste gemeint, die an das oder die Stickstoffatome des Aminoalkohols oder Diamins mit jeweils (zumindest) einer sterisch gehinderten sekundären Aminogruppe gebunden sind, d. h. sowohl des die beiden Aminogruppen bzw. die Amino- und die Hydroxylgruppe verbindenden zweiwertigen Rests $R_1$ als auch des Substituenten $R_2$ am Stickstoffatom der sekundären Aminogruppe(n), wie in den nachstehenden Formeln (II) (Diamin) und (III) (Aminoalkohol) dargestellt ist:

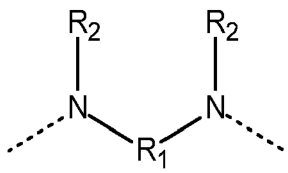
(II)

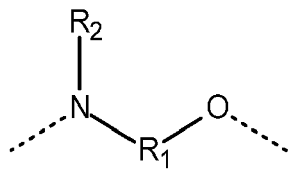
(III)

worin die gestrichelten Linien jeweils die Bindung an die Carbonylgruppe einer die Reste I, M, $C_1$ und $C_2$ verbindenden Urethan- oder Harnstoffgruppierung anzeigen und zumindest einer der Reste $R_1$ und $R_2$ ein sperriger Rest ist, der eine sterische Hinderung der Harnstoffgruppierung innerhalb des TPUUs, deren Teil das jeweilige Stickstoffatom ist, bewirkt. Im Falle von Diaminen mit nur einer sekundären Aminogruppe ist einer Reste $R_2$ in Formel (II) Wasserstoff, und im Falle von Diaminen mit zwei sterisch gehinderten sekundären Aminogruppen ist in Formel (II) entweder zumindest der Rest $R_1$ (und gegebenenfalls auch einer oder beide der Reste $R_2$) ein sperriger Rest, oder beide Reste $R_2$ (und gegebenenfalls auch der Rest $R_1$) sind sperrige Reste.

In bevorzugten Ausführungsformen sind in den TPUUs der Formel (I) die Reste $C_1$ jeweils unabhängig von einem Diamin abgeleitet und aus Resten der nachstehenden Formel (II) ausgewählt:

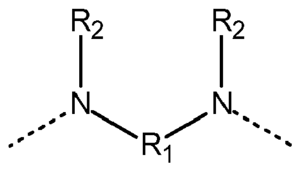

(II)

worin $R_1$ aus zweiwertigen, gesättigten oder ungesättigten, aliphatischen oder alicyclischen Resten mit 1 bis 20 Kohlenstoffatomen ausgewählt ist; und

die $R_2$ jeweils unabhängig aus Wasserstoff sowie einwertigen, sperrigen, gesättigten oder ungesättigten, aliphatischen oder alicyclischen Resten mit 1 bis 10 Kohlenstoffatomen ausgewählt sind, mit der Maßgabe, dass nicht beide $R_2$ gleichzeitig Wasserstoff sind;

noch bevorzugter aus Resten der obigen Formel (II), worin

$R_1$ aus zweiwertigen, gesättigten oder ungesättigten, aliphatischen oder alicyclischen Resten mit 1 bis 10 Kohlenstoffatomen ausgewählt ist; und/oder

die $R_2$ jeweils unabhängig aus einwertigen, sperrigen, gesättigten oder ungesättigten, aliphatischen oder alicyclischen Resten mit 1 bis 10 Kohlenstoffatomen ausgewählt sind;

besonders bevorzugt aus Resten der Formel (II), worin

$R_1$ aus $C_1$-$C_{10}$-Alkylen- und $C_4$-$C_{10}$-Cycloalkylenresten ausgewählt ist; und/oder

die $R_2$ jeweils unabhängig aus 1,1-Dimethyl-substituierten, gesättigten oder ungesättigten $C_1$-$C_6$-Alkylresten und 1-Methyl-substituierten $C_3$-$C_6$-Cycloalkylresten ausgewählt sind;

und insbesondere aus Resten der Formel (II), worin

$R_1$ aus $C_2$-$C_6$-Alkylen- und $C_5$-$C_6$-Cycloalkylenresten ausgewählt ist; und/oder

die $R_2$ jeweils unabhängig aus Isopropyl, tert-Butyl, 1,1-Dimethylpropyl und 1-Methylcyclohexyl ausgewählt sind.

Diese bevorzugten Auswahloptionen aus diesen physiologisch unbedenklichen, relativ kurzkettigen Resten beidseitig sterisch gehinderter sekundärer Diamine bewirken, dass im Vergleich zu den höhermolekularen Weichsegmenten M kurzkettige Hartsegmente $C_1$ in den TPUUs der Formel (I) enthalten sind, was deren Thermoplastizität fördert und in der Folge auch die Wahrscheinlichkeit der "Rekombinationsreaktion" gemäß obigem Schema D während des Kontakts der daraus elektrogesponnenen Gefäßprothesen mit Wasser erhöht.

Weiters umfasst in bevorzugten Ausführungsformen zumindest einer der Reste $C_2$ der TPUUs eine oder mehrere Estergruppierungen, die besonders bevorzugt jeweils unabhängig von einem Diol aus der aus den folgenden bestehenden Gruppe abgeleitet sind: Bis(hydroxyethyl)terephthalat, Bis(hydroxypropyl)carbonat, 2-Hydroxyethyllactat und 2-Hydroxyethylglykolat, da diese relativ kurzkettig und physiologisch unbedenklich sind.

Besonders bevorzugt ist in den TPUUs der Formel (I) b + 1 = a + c, so dass das Polyaddukt der nachstehenden Formel (IV) entspricht:

$$-[(I\text{-}M\text{-}I\text{-}C_1)_a\text{-}(I\text{-}M\text{-}I\text{-}C_2)_c]_n- \qquad (IV)$$

worin

a und c jeweils unabhängig aus 1 bis 3 ausgewählt sind oder

a und c jeweils 1 sind; und

$n \geq 5$ oder $n \geq 10$ oder $n \geq 20$ ist.

Dadurch ist vor allem das Polyadditionsverfahren zur Herstellung der TPUUs gut steuerbar, wobei vorzugsweise zunächst das gewünschte Makrodiol (oder auch mehrere verschiedene) in Lösung in einem geeigneten wasserfreien organischen Lösungsmittel mit etwas mehr als der doppelten molaren Menge an Diisocyanat umgesetzt wird, um beidseitig Isocyanat-terminierte Präpolymere oder Zwischenprodukte mit der Kettenstruktur I-M-I zu erhalten, wonach die beiden, die Kettenverlängerer $C_1$ und $C_2$ einführenden Reaktanten nacheinander in molaren Mengen zugesetzt werden, deren Summe der molaren Menge des Makrodiols entspricht.

Aufgrund der geringeren Reaktivität der sterisch gehinderten Amine und der Instabilität der von ihnen gebildeten Harnstoffbindungen, vor allem in Lösung, wird im Polyadditionsverfahren die Kettenverlängerer-Gruppierung $C_1$ vorzugsweise als letzter Baustein in die TPUUs eingeführt. Somit wird das Isocyanat-terminierte Präpolymere mit der Kettenstruktur I-M-I vorzugsweise zunächst mit dem $C_2$ enthaltenden Diol, Diamin oder Aminoalkohol ohne sterisch

gehinderte sekundäre Aminogruppen umgesetzt, was beispielsweise bei Reaktion der in Bezug auf die Makrodiol-Einheiten M halben molaren Menge an Kettenverlängerer Isocyanat-terminierte Zwischenprodukte mit der Kettenstruktur I-M-I-$C_2$-I-M-I ergibt. Diese werden anschließend mit dem $C_1$ enthaltenden Kettenverlängerer-Reagens, d. h. dem sterisch gehinderte Aminogruppen enthaltenden Diamin oder Aminoalkohol, umgesetzt, was bei äquimolaren Mengen der beiden Kettenverlängerer ein TPUU-Polymer mit Blöcken einer Struktur der Formel (V) ergibt:

$$-[I\text{-}M\text{-}I\text{-}C_1\text{-}I\text{-}M\text{-}I\text{-}C_2]_n- \qquad (V)$$

d. h. TPUUs der Formel (I), worin a = b = c = 1 ist. Der Wert von n, d. h. die Anzahl an Blöcken, und damit die Kettenlänge und das Molekulargewicht der TPUUs hängen dabei neben der Reinheit der Monomere, wie zuvor erwähnt, vor allem von deren Stöchiometrie sowie von der Reaktionsführung und dabei vor allem von der Reihenfolge des Zusatzes der verschiedenen Monomer- oder Präpolymer-Komponenten ab. Vorzugsweise ist $n \geq 5$, noch bevorzugter $\geq 10$, noch bevorzugter $\geq 20$.

In weiteren bevorzugten Ausführungsformen ist die vorliegende Erfindung dadurch gekennzeichnet, dass in dem Elektrospinn-Verfahren eine Lösung des TPUU in einem organischen Lösungsmittel oder Lösungsmittelgemisch, besonders bevorzugt eine Lösung in Hexafluorisopropanol, in einer Elektrospinnvorrichtung, die einen Hochspannungsgenerator, eine Spritzenpumpe, eine Spritze mit stumpfem Ende als Elektrode, einen geerdeten, elektrisch leitenden rotierenden Stahldorn als Kollektorelektrode und gegebenenfalls eine Hilfselektrode umfasst, mittels der Spritze in das zwischen den Elektroden aufgebaute elektrische Feld injiziert und die sich dabei bildenden Polymerfasern als Endlos-Nanofasern auf dem rotierenden Dorn zu einer als Gefäßprothese geeigneten Röhre aufgewickelt werden. Eine solche Verfahrensführung hat sich bereits in der Vergangenheit bei der Herstellung von Gefäßprothesen bewährt.

In besonders bevorzugten Ausführungsformen wird eine Lösung eines Gemischs aus dem TPUU der Formel (I) und zumindest einem weiteren Polymer eingesetzt und werden beim Elektrospinnen aus dem Gemisch bestehende Röhren hergestellt. Es hat sich bei den Experimenten der Erfinder nämlich herausgestellt, dass bei der Herstellung von Prothesen, die aus dem TPUU der Formel (I) alleine bestehen, die Faserbildung weniger gut funktioniert und dass die Porosität des Materials geringer ist. Aus letzterem Grund ist die Zugänglichkeit der aufgewickelten Fasern für wässrige Medien wie Blut eingeschränkt, was die biologische Abbaubarkeit der Prothese verringert. Das zumindest eine weitere Polymer wird dabei vorzugsweise in einem Anteil von zumindest 10 Gew.-%, zumindest 15 Gew.-%, zumindest 20 Gew.-%, zumindest 25 Gew.-%, zumindest 30 Gew.-% oder gegebenenfalls auch zumindest 50 Gew.-%, des Gemischs eingesetzt, um die obigen Eigenschaften des Gemischs zu verbessern.

Weiters wird gemäß vorliegender Erfindung vorzugsweise ein Gemisch aus dem TPUU der Formel (I) und einem TPU eingesetzt, noch bevorzugter einem biologisch abbaubaren TPU, das sich in der Vergangenheit bereits als für die Herstellung von Gefäßprothesen geeignet erwiesen hat, wobei gemäß vorliegender Erfindung insbesondere ein Polyetherurethan, wie z.B. ein Polyaddukt aus Polytetrahydrofuran, Bis-(hydroxyethyl)terephthalat und Hexamethylendiisocyanat, als TPU im Gemisch mit dem TPUU der Formel (I), z.B. ein Gemisch aus jeweils 50 Gew.-% TPUU und TPU, eingesetzt wird.

In einem zweiten Aspekt stellt die vorliegende Erfindung auch die durch das erfindungsgemäße Elektrospinn-Verfahren des ersten Aspekts unter Verwendung von TPUUs der Formel (I) erhältlichen Gefäßprothesen bereit, die sich durch gute Verarbeitbarkeit und biologische Abbaubarkeit auszeichnen, wie die späteren Beispiele belegen.

## KURZBESCHREIBUNG DER ZEICHNUNGEN

Die vorliegende Erfindung wird nachstehend anhand von illustrativen, nichteinschränkenden Ausführungsbeispielen und unter Bezugnahme auf die beiliegenden Zeichnungen näher beschrieben, die Folgendes zeigen:

In Fig. 1 sind zwei Mikroskopaufnahmen (Fig. 1a und 1b) der Prothese aus Beispiel 1 der vorliegenden Erfindung dargestellt.

Fig. 2 zeigt ein Foto (Fig. 2a) sowie zwei Mikroskopaufnahmen (Fig. 2b und 2c) der Prothese aus Beispiel 2 der vorliegenden Erfindung.

Fig. 3 und Fig. 4 sind grafische Darstellungen der Ergebnisse von Zugfestigkeitstests mit den Prothesen aus Beispiel 1 und Beispiel 2.

Die Fig. 5 bis 7 sind grafische Darstellungen der Ergebnisse von Tests zur biologischen Abbaubarkeit des in den Prothesen enthaltenen TPUU.

Die Fig. 8 und 9 zeigen die Ergebnisse von Tests der Zytotoxizität des TPUU für HUVECs bzw. Makrophagenzellen

(Fig. 8a und 8b) bzw. des zu dessen Herstellung eingesetzten Monomers TBEDA für HUVECs (Fig. 9).

Die Fig. 10a und 10b sind Mikroskopaufnahmen während Tests bezüglich der Adhäsion und Proliferation von EPCs auf der Prothese aus Beispiel 1.

Und die Fig. 11A-H sind Fotografien verschiedener Stadien von Biokompatibilitätstests mit der Gefäßprothese aus Beispiel 2 in vivo.

BEISPIELE

Als repräsentative Beispiele für besonders bevorzugte Ausführungsformen der vorliegenden Erfindung wurden ein TPUU sowie ein Gemisch aus dem TPUU und einem TPU mittels Elektrospinnen zu Röhren aus Endlosfasern verarbeitet, die in der Folge Tests auf ihre Eignung als Gefäßprothesen unterzogen wurden. Davor wurde zunächst das TPUU unter Anwendung der zuvor dargelegten bevorzugten Reaktionsführung hergestellt, d. h. durch aufeinanderfolgende Reaktion der einzelnen Komponenten unter anfänglicher Herstellung von beidseitig Isocyanat-terminierten Präpolymeren bzw. Zwischenprodukten mit der Kettenstruktur I-M-I, die nacheinander mit den beiden, die Kettenverlängerer $C_2$ und danach $C_1$ einführenden Reaktanten umgesetzt wurden. Das TPU entspricht "TPU4" der in Baudis et al. (2012; s.o.) offenbarten thermoplastischen Polyetherurethane und wurde aus Polytetrahydrofuran, Bis(hydroxyethyl)terephthalat und Hexamethylendiisocyanat hergestellt. Beide Synthesen werden in den nachfolgenden Synthesebeispielen detaillierter beschrieben.

Svnthesebeispiel 1

Unter Anwendung der Standard-Schlenktechnik mit Argon als Inertgas wurde zunächst vorgetrocknetes Polytetrahydrofuran (pTHF) ($M_n \approx$ 1 kDa, 6,059 g, 6,1 mmol, 1,00 Äqu., 19 ppm $H_2O$) als Makrodiol in einen Reaktionskolben eingewogen und eine weitere Stunde lang bei 60 °C im Hochvakuum getrocknet. Anschließend wurden 5 ml abs. Dimethylformamid (DMF) und danach Hexamethylendiisocyanat (HMDI) (2,111 g, 12,6 mmol, 2,07 Äqu.) in 5 ml abs. DMF zu dem trockenen, geschmolzenen pTHF zugesetzt. Nach der Zugabe von 2 Tropfen (ca. 0,04 ml) Zinn(II)-2-ethylhexanoat als Katalysator wurde das Reaktionsgemisch unter Argon-Schutzatmosphäre 3 h lang bei 60 °C magnetisch gerührt. Anschließend wurde Bis(hydroxyethyl)terephthalat (BHET) (0,770 g, 3,03 mmol, 0,5 Äqu.) als Diol zur Einführung von $C_2$ in Form einer Lösung in 5 ml abs. DMF zugesetzt. Nach weiteren 3 h Rühren bei 60 °C wurde das Reaktionsgemisch auf Raumtemperatur abgekühlt, wonach N,N'-Di-tert-butylethylendiamin (TBEDA) (0,522 g, 3,03 mmol, 0,5 Äqu.) als beidseitig sterisch gehindertes sekundäres Diamin zur Einführung von $C_1$ zugesetzt wurde. Nach jeder Zugabe wurden die Transfer-Gefäße bzw. -Spritzen jeweils mit 5 ml abs. DMF gespült. Die Reaktionslösung wurde über Nacht weitergerührt. Zur Gewinnung und Reinigung des hergestellten TPUU wurde das Reaktionsgemisch mit DMF verdünnt und zum zehnfachen Volumen an Diethylether zugetropft und dabei als farbloser Niederschlag ausgefällt, der anschließend getrocknet und mittels GPC und NMR charakterisiert wurde.

Durch diese Umsetzung der Reaktanten im Verhältnis $C_1 : C_2 : M : I = 1 : 1 : 2 : 4$ (bzw. 4,14) wurde ein TPUU der obigen Formel (IV) erhalten, worin a = b = c = 1 ist, d. h. ein TPUU der Formel (V):

$$-[I-M-I-C1-I-M-I-C2]_n- \qquad (V)$$

Der Wert für n wurde aus dem mittels Gelpermeationschromatographie (GPC) bestimmten gewichtsmittleren Molekulargewicht ($M_w$) und der Molmasse der Blöcke berechnet. Für $M_w$ des erhaltenen TPUU wurden rund 65,6 kDa ermittelt, und die Molmasse eines Blocks betrug rund 3,1 kDa, woraus ein Wert für n von rund 21 folgt.

Die genaue Struktur dieses TPUU der Formel (V) ist umseitig dargestellt. Der durchschnittliche Wert für m der von PolyTHF mit einem zahlenmittleren Molekulargewicht $M_n$ von etwa 1 kDa stammenden Einheiten M beträgt darin rund 14. Unterhalb sind weiters die den Einheiten $C_1$, $C_2$, M und I entsprechenden Abschnitte angegeben.

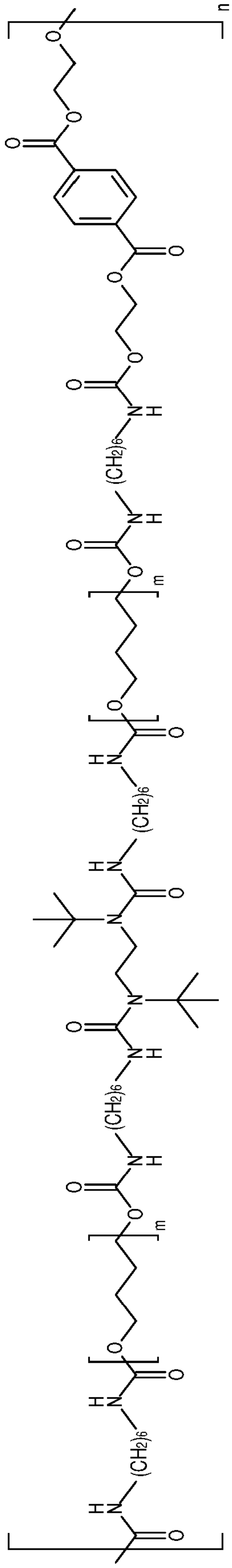

$C_2$

I

M

I

$C_1$

I

M

I

Svnthesebeispiel 2

Auf analoge Weise zu Synthesebeispiel 1 wurde ein TPU durch Umsetzung von pTHF ($M_n \approx 1$ kDa), HMDI und BHET hergestellt, wobei jedoch kein sterisch gehindertes sekundäres Diamin zur Einführung von $C_1$ zugesetzt, sondern eine zur Menge an pTHF äquimolare Menge an BHET eingesetzt wurde. Als Resultat beträgt das Verhältnis der Reste im Polyaddukt $C_2$ : M : I = 1 : 1 : 2, das somit ein thermoplastisches Polyurethan (TPU; ohne Harnstoffgruppierungen) der nachstehenden Formel (VI) darstellt:

$$-[I\text{-}M\text{-}I\text{-}C2]_n- \qquad (VI)$$

Für das $M_w$ des so erhaltenen TPU wurden mittels GPC 46 kDa ermittelt, und die Molmasse eines Blocks betrug rund 1,6 kDa, woraus ein Wert für n von rund 29 folgt.

Dieses TPU ist zwar aufgrund der spaltbaren Esterbindungen in $C_2$ unter physiologischen Bedingungen abbaubar, weist aber natürlich keine selbstverstärkenden Eigenschaften auf.

**Beispiele 1 und 2 - Herstellung von Gefäßprothesen durch Elektrospinnen**

Beispiel 1 - TPUU

Das in Synthesebeispiel 1 hergestellte TPUU wurde in Hexafluorisopropanol gelöst und unter Einsatz einer Elektrospinnvorrichtung, die einen Hochspannungsgenerator, eine Spritzenpumpe, eine Spritze mit einer 21-G-Nadel mit stumpfem Ende als Auslassdüse, einen geerdeten, elektrisch leitenden rotierenden Stahldorn als Kollektorelektrode und eine Hilfselektrode umfasste, zu einer Endlos-Nanofasern verarbeitet, die auf dem rotierenden Dorn zu einer Röhre aufgewickelt wurden. Die Potentialverteilungen zwischen den einzelnen Elektroden waren dabei einzeln einstellbar, und die Potentiale des rotierenden Dorns und der Hilfselektrode wurden so eingestellt, dass eine optimale Faserabscheidungsrate erzielt wurde. Der Abstand zwischen der Nadelspitze und dem Dorn betrug 8 cm. Die Elektrospinnvorrichtung wurde als Ganzes in einen Faradayschen Käfig gestellt und in einem Reinraum der Klasse 1000 bei einer Temperatur von 24 °C und 34 % r. L. betrieben, wobei der Zufluss der Polymerlösung in die Spritze an der Spritzenpumpe auf 0,7 ml/h eingestellt und an dem aus der Nadelspitze austretenden Polymer eine Spannung von 12 kV angelegt wurde.

Beispiel 2 - TPUU und TPU

Eine Lösung eines Gemischs aus jeweils 50 Gew.-% des TPUU aus Synthesebeispiel 1 und des TPU aus Synthesebeispiel 2 in Hexafluorisopropanol wurde auf analoge Weise wie in Beispiel 1 zu elektrogesponnenen Röhren verarbeitet, wobei jedoch an dem aus der Nadelspitze austretenden Polymergemisch eine Spannung von 8,5 kV angelegt wurde.

Die so auf dem Dorn erhaltenen elektrogesponnenen Röhren beider Beispiele wiesen einen Innendurchmesser von 1,5 mm und eine Wandstärke von etwa 300 μm auf und wurden zur Entfernung von Restlösungsmittel jeweils 2 h lang im Vakuum bei 40 °C getrocknet.

**Testung der physikalischen und biomechanischen Eigenschaften**

Die in Beispiel 1 und Beispiel 2 erhaltenen trockenen Röhren wurden jeweils zu Ringen mit einer Breite von 2 mm geschnitten auf die folgende Weise auf ihre Eigenschaften untersucht.

Faserbildung, Porosität

Zur Oberflächencharakterisierung der Prothesen aus Beispiel 1 und Beispiel 2 wurden die Innen- und Außenflächen mit jeweils Gold-Palladium beschichtet. Die luminale Morphologie wurde mit einem Rasterelektronenmikroskop EVO 10 von Zeiss, Deutschland, bei einer Beschleunigungsspannung von 10 kV mit 3500facher Vergrößerung betrachtet. Die Faserstruktur an der Innen- und der Außenfläche der Gefäßprothese aus Beispiel 1 sind in Fig. 1a bzw. 1b dargestellt, und Fig. 2 zeigt Ansichten der Faserstruktur an der Außen- (Fig. 2a), der Querschnitts- (Fig. 2b) und der Innenfläche (Fig. 2c) der Prothese aus Beispiel 2.

Aus den Fig. 1a und 1b ist zu erkennen, dass es bei alleiniger Verwendung des TPUU aus Synthesebeispiel 1 zur Herstellung der Gefäßprothesen in Beispiel 1 beim Elektrospinnen zu keiner optimalen Faserbildung kam, was sich anhand von relativ geringer Porosität der gesponnenen Röhren zeigt. Beim Elektrospinnen des 50:50-Gemischs aus dem TPUU und einem herkömmlichen TPU in Beispiel 2 wurde hingegen die eine Fig. 2a abgebildete Prothese aus einem Faservlies mit gut ausgeprägten einzelnen Fasern und gut definierten Leerstellen dazwischen erhalten, wodurch

diese gegenüber jener aus Beispiel 1 eine deutlich verbesserte Porosität aufweist. Letztere erhöht nach späterer Implantation der Prothesen die Zugänglichkeit der einzelnen Fasern für Blut und verbessert somit deren biologische Abbaubarkeit im Zeitverlauf.

Zugfestigkeit

*A) Prothesen aus Beispiel 1*

Von den 2 mm breiten Ringen aus den in Beispiel 1 erhaltenen Prothesen wurden fünfzehn Stück bis zu 34 d lang bei Raumtemperatur trocken bzw. in physiologische Kochsalzlösung eingelegt gelagert.

Nach 1, 2, 7, 14 und 34 d Lagerung wurden mit jeweils drei nass bzw. trocken gelagerten Ringen Zugversuche durchgeführt, indem diese unter Verwendung einer ElectroForce® TestBench von Bose in Umfangsrichtung mit einer Geschwindigkeit von 10 mm/min bis zum maximalen Verfahrweg der Maschine (12 mm) gedehnt wurden. Die Tests wurden in einem Premiere Tissue Floating Bath XH-I 003 bei 37 °C durchgeführt, wobei mittels WinTest-Software Kraft-Dehnungs-Kurven aufgezeichnet und mit einer Analysetabelle auf Matlab-Basis ausgewertet wurden.

Fig. 3 zeigt einen Vergleich der so erhaltenen Ergebnisse für die trocken gelagerten und die wasserbehandelten Ringe. Man erkennt, dass bereits nach nur 1 d die als maximal anwendbare Kraft (in N) angegebene Zugfestigkeit der wasserbehandelten Ringe im Mittel um gut ein Drittel höher war als jene der trocken gelagerten. Dieser Effekt verstärkte sich bei weiterer Lagerung noch weiter, bis nach 7 d die wasserbehandelten Ringe knapp die dreifache Reißfestigkeit der trocken gelagerten aufwiesen. Waren bis dahin noch sämtliche Ringe vor Erreichen des im vorliegenden Fall maximalen Verfahrweges von 12 mm gerissen, so rissen ab Tag 7 die wasserbehandelten Ringe nicht mehr. Bei den späteren Tests nach 14 d bzw. 34 d wurden jeweils im Wesentlichen die gleichen Werte wie nach 7 d festgestellt.

*B) Prothesen aus Beispiel 2*

Auf Basis der oben mit den Prothesen aus Beispiel 1 erzielten Ergebnisse wurden die Ringe aus den in Beispiel 2 erhaltenen Prothesen jeweils 7 d lang bei Raumtemperatur trocken bzw. in physiologische Kochsalzlösung eingelegt gelagert. Anschließend wurde analog zu oben die maximale Zugkraft bis zum Reißen der Ringe unter Verwendung der ElectroForce® TestBench von Bose gemessen. Die Ergebnisse sind als Mittelwerte plus Standardabweichung in Fig. 4 grafisch dargestellt.

Aus der Grafik ist ersichtlich, dass die trocken gelagerten Ringe im Mittel einer Maximalkraft von 0,98 N, die nass gelagerten jedoch 1,55 N standhielten. Somit wurde auch die Zugfestigkeit des Polymergemischs aus TPUU und TPU durch die 7-tägige Lagerung um rund die Hälfte verbessert, was den selbstverstärkenden Effekt bei Kontakt mit einem wässrigen Milieu auch für dieses Material belegt.

Aus der Tatsache, dass bei 50%igem Zusatz des TPU ohne selbstverstärkende Wirkung die Erhöhung der Zugfestigkeit rund 50 % jener der Ringe aus dem TPUU alleine betrug, kann geschlossen werden, dass hierbei möglicherweise ein linearer Zusammenhang besteht. Jedenfalls ist anzunehmen, dass ein geringerer Anteil an TPU im Gemisch mit dem TPUU, z.B. nur 25 Gew.-%, eine stärkere und ein höherer Anteil, z.B. 75 Gew.-%, eine geringere Erhöhung der Zugfestigkeit der jeweiligen Prothesen bewirken kann. Die Herstellung von Prothesen aus solchen Materialgemischen ist derzeit Gegenstand weiterer Experimente der Erfinder.

**Testung der Abbaubarkeit**

Das in Synthesebeispiel 1 erhaltene TPUU wurde in einer Konzentration von 10 Gew.-% in abs. DMF gelöst. Diese Lösung wurde in Teflon-Formen mit den Abmessungen 60 x 40 x 2 mm gegossen, und das Lösungsmittel wurde bei Raumtemperatur abdampfen gelassen. Nach 24 h wurde die so erhaltenen Folie weitere 3 d lang im Exsikkator unter Vakuum getrocknet, wonach ihre Dicke mittels eines elektronischen Außenmessgeräts K110T von Kroeplin gemessen wurde und jeweils rund 800 μm betrug. Aus dieser Folie wurden jeweils 15 kreisförmige Scheiben mit einem Durchmesser von 5 mm ausgestanzt, deren Gewicht genau bestimmt wurde, das jeweils zwischen 15 und 20 mg betrug.

Anschließend wurde als Simulation physiologischer Bedingungen jeweils eine Scheibe in einem Reagenzglas in 20 ml PBS (1X, pH 7,4) eingelegt, wonach die Reagenzgläser in einem Autoklaven auf 90 °C erhitzt wurden. Nach 7, 14, 25, 35 und 41 d wurden jeweils drei davon entnommen. Die darin enthaltenen Scheiben wurden je dreimal für 15 min in entionisiertes Wasser eingelegt, um die enthaltenen Salze zu entfernen. Anschließend wurden sowohl das Abtropfgewicht als auch - nach Trocknung bis zur Gewichtskonstanz (24 h bei 80 °C und 120 mbar) - das Trockengewicht bestimmt sowie eine Molekulargewichtsbestimmung mittels Gelpermeationschromatographie durchgeführt. Aus den so erhaltenen Werten wurden anhand der nachstehenden Gleichungen 1 bis 3 der Massenverlust, die Molekulargewichtsabnahme und die Quellung der einzelnen Proben berechnet.

Gleichung 1: $$m_{eros}(t) = \frac{m_t - m_0}{m_0} \cdot 100$$

Gleichung 2: $$\%\overline{M}_w(t) = \frac{\overline{M}_w(t)}{\overline{M}_w(0)} \cdot 100$$

Gleichung 3: $$s(t) = \frac{m_t^w - m_t}{m_t} \cdot 100$$

$m_{eros}(t)$ Massenverlust nach t Tagen Abbau
t Abbau-Dauer in d
$m_t$ Probengewicht nach t Tagen Abbau in mg
$m_0$ Probengewicht vor dem Abbau in mg
$\%\overline{M}_w(t)$ Molekulargewichtsabnahme nach t Tagen Abbau in %
$\overline{M}_w(t)$ Molekulargewicht nach t Tagen Abbau in kDa
$\overline{M}_w(0)$ Molekulargewicht vor dem Abbau in kDa
s(t) Quellung der Probe nach t Tagen Abbau in %
$m_t^w$ Abtropfgewicht der Probe nach t Tagen Abbau in mg

Die Ergebnisse dieser Berechnungen sind in den Fig. 5 bis 7 grafisch dargestellt.

Daraus lässt sich vor allem ablesen, dass bereits nach 7 d eine Molekulargewichtsabnahme von rund 3/4 des Ausgangs-Molekulargewichts - bei rund 10%igem Massenverlust und kaum erhöhter Quellbarkeit - erfolgt war, die bis zum Ende der Abbaustudie nach 41 d - genau wie der Massenverlust - allmählich auf rund 90 % angestiegen war. Aus einer Extrapolation der Graphen der Fig. 5 und 6 lässt sich schließen, dass der Abbau nach etwa einer weiteren Woche bei 90 °C in PBS, d. h. nach insgesamt rund 7 Wochen, wohl vollständig gewesen wäre.

Das zur gemäß vorliegender Erfindung zur Herstellung von Gefäßprothesen verwendete TPUU der Formel (V) ist somit binnen weniger Wochen vollständig hydrolytisch abbaubar und unter diesem Aspekt für diesen Zweck hervorragend geeignet.

**In-vitro-Biokompatibilitätsprüfung**

A) Herstellung von Makrophagen-, EPC- und HUVEC-Kulturen

Für die Kultur von primären Makrophagen und Endothel-Vorläuferzellen (EPCs) wurde frisch gespendetes Blut von gesunden humanen Spendern (45 ml) durch Dichtezentrifugation mit Ficoll Paque (GE Healthcare, USA) 30 min lang bei 300 g ungebremst in verschiedene Zellpopulationen getrennt. Der Buffy Coat mit peripheren mononukleären Blutzellen (PBMCs) wurde vorsichtig in ein frisches Zentrifugenröhrchen pipettiert, mit Phosphatpuffer-Salzlösung (PBS, Sigma-Aldrich) auf 50 ml aufgefüllt und 10 min lang bei 500 g zentrifugiert, um die Zellen zu waschen und Thrombozyten zu entfernen. Dieser Schritt wurde einmal wiederholt. Die EPCs wurden anschließend für Besiedelungsversuche der 2 mm breiten Ringe verwendet. Um Makrophagen zu differenzieren, wurden die Zellen in RPMI 1640-Medium (ergänzt mit 10% FBS, Sigma-Aldrich, USA) resuspendiert, gezählt und in T175-Zellkulturflaschen (50 x $10^6$ Zellen pro Flasche) für 2 h im Inkubator bei 37 °C ausgesät. Nach 2 h wurde das Zellkulturmedium entfernt, die Zellen wurden zweimal sorgfältig mit PBS gewaschen, und RPMI 1640 mit 50 ng/ml M-CSF (Makrophagen-Kolonie-stimulierender Faktor) wurde zugegeben. Nach 3 d wurde ein weiterer Mediumwechsel mit M-CSF durchgeführt, und die Zellen wurden am nächsten Tag für Experimente verwendet.

Humane Nabelvenen-Endothelzellen (HUVEC, gepoolter Spender, Lonza, Schweiz) wurden in endothelialem Wachstumsmedium (EGM-2, Lonza, Schweiz) kultiviert, das mit 10 % FBS ergänzt wurde. Der Mediumwechsel wurde jeden zweiten Tag durchgeführt. Die Zellen wurden zwischen den Passagen 3 und 6 für die Experimente verwendet.

B) Zytotoxizitätstests mit HUVEC- und Makrophagenkulturen

XTT (2,3-Bis(2-methoxy-4-nitro-5-sulfophenyl)-5-[(phenylamino)carbonyl)-2H-tetrazo-liumhydroxid) kann in lebensfähigen Zellen zu einem Formazan-Produkt reduziert werden. Das Ergebnis kann durch photometrische Messungen bestimmt werden und korreliert mit der Anzahl der lebensfähigen Zellen. Der Assay wurde auf der Grundlage von ISO10993-5 durchgeführt.

*B1 - Polymervergleich*

Wie oben beschrieben wurde aus dem in Synthesebeispiel 1 erhaltenen TPUU Folien gezogen, aus denen kreisförmige Scheiben mit einem Durchmesser von 5 mm ausgestanzt wurden ("TPUU"). Zum Vergleich wurden auf die gleiche Weise Scheiben aus dem in Synthesebeispiel 2 erhaltenen TPU ("TPU1"), aus Pellethane™ 2363-80A, einem handelsüblichen TPU von Lubrizol Inc. aus Methan-4,4'-diphenyldiisocyanat (MDI), Polytetrahydrofuran (pTHF) und 1,4-Butandiol (BDO) ("TPU2"), sowie einem in Ehrmann et al. (2020; s.o.) beschriebenen TPU, nämlich einem Polycarbonat-Urethan aus Polyhexamethylencarbonat (pHMC), Hexamethylendiisocyanat (HMDI) und Bis(3-hydroxypropylen)carbonat (BHPC) ("TPU3") hergestellt. Diese drei TPUs hatten sich in der Vergangenheit bereits bei der Synthese von Gefäßprothesen bewährt.

HUVECs oder Makrophagen wurden auf den Polymerscheiben in einer Zellanzahl von 10.000 Zellen/Scheibe in einer 96-Well-Platte für 24 h ausgesät, bis sich eine konfluente Zellschicht gebildet hatte. XTT-Pulver wurde in 60 °C heißem Zellkulturmedium in einer Konzentration von 1 mg/ml gelöst. PMS (Phenazinmethosulfat, Sigma-Aldrich, USA) wurde in PBS in einer Konzentration von 5 mM gelöst. Zur Herstellung einer Arbeitslösung, die den Zellen zugesetzt wurde, wurde PMS in einer Konzentration von 25 μM in die XTT-Lösung pipettiert. Die Zellen wurden mit 50 μl dieser XTT-Arbeitslösung 4 h lang bei 37 °C im Zellkultur-Brutschrank inkubiert. Das Medium wurde dann auf eine neue 96-Well-Platte übertragen, und die Absorption bei 450 nm wurde mit einer Referenzwellenlänge von 620 nm gemessen. Die Ergebnisse sind als Mittelwerte von jeweils drei Bestimmungen in Fig. 8 dargestellt.

Aus den Grafiken ist zu erkennen, dass für die vier untersuchten Polymere keine allzu großen Unterschiede bei der Lebensfähigkeit von HUVECs und Makrophagen festzustellen waren. Auch wenn im Falle der Makrophagenkultur das TPUU aus Synthesebeispiel 1 am schlechtesten abgeschnitten hatte, so liegt die Zytotoxizität aller vier untersuchten Polymere doch in derselben Größenordnung, was die Eignung des TPUU für die Herstellung von Gefäßprothesen ein weiteres Mal belegt.

*B2 - Monomertest*

Um die Zytotoxizität des im TPUU enthaltenen Monomers N,N'-Di-tert-butylethylendi-amin (TBEDA) zu bewerten, das in Beispiel 1 hierin erstmalig zur Herstellung einer Gefäßprothese eingesetzt wurde, wurden HUVECs auf 96-Well-Platten ausgesät und kultiviert, bis 80%ige Konfluenz erreicht war. TBEDA wurde in Form einer Lösung in Dimethylsulfoxid (DMSO) so zugesetzt, dass eine Konzentration von 450 μmol/ml erzielt wurde, wovon in der Folge eine Verdünnungsreihe mit dem Verdünnungsfaktor 10 erstellt wurde. Die Lösungen wurden anschließend allesamt 24 h lang inkubiert, wonach der XTT-Test wie oben beschrieben durchgeführt wurde, dessen Ergebnisse als Mittelwerte von Vierfachbestimmungen in Fig. 9 dargestellt sind. Darin ist zu erkennen, dass schon ab einer TBEDA-Konzentration von 45 μmol/ml die Lebensfähigkeit der HUVECs auf einem akzeptablen Niveau lag.

C) Adhäsion und Proliferation von humanen Endothel-Vorläuferzellen

Wie oben beschrieben wurden PBMCs aus frischem humanen Blut drei verschiedener Spender isoliert und in einer Zellanzahl von 2 Mio Zellen pro ml auf 2 mm breiten Ringen aus dem in Beispiel 1 elektrogesponnenen TPUU sowie auf expandiertem Polytetrafluorethylen (ePTFE) als Kontrolle gesiedelt. Nach zwei Wochen Inkubation mit dreimaligen Mediumwechseln pro Woche wurden die Zellen mit 4% Paraformaldehyd über Nacht bei 4 °C fixiert und anschließend nach mehrmaligem Waschen mit PBS mit dem EPC-spezifischen Antikörper gegen Pro1 (Invitrogen, USA, Kat.-Nummer: MA1-219) 45 min lang bei Raumtemperatur gefärbt. Nach zwei erneuten Waschschritten mit PBS wurde mit dem sekundären, Alexa Fluor 647 konjugierten Antikörper (Abcam, Kat.-Nummer: ab150115) ebenfalls für 45 min bei Raumtemperatur inkubiert. Nach einem finalen Waschschritt mit PBS wurden die Proben mithilfe von Eindeckmedium auf Mikroskopie-Gläser aufgebracht, und Fotos der Zellen wurden mit einem LSM-700 Konfokalmikroskop (Zeiss, Deutschland) aufgenommen. Diese sind in Fig. 10 abgebildet.

Man erkennt, dass die Anzahl an adhärierten EPCs auf der Prothese aus Beispiel 1 im Vergleich zu ePTFE deutlich erhöht ist, wenn auch zwischen den verschiedenen Spendern relativ starke Unterschiede in der Prom1-Expression und der adhärierten Zellanzahl auftreten. Das in Beispiel 1 verwendete TPUU wurde jedenfalls in signifikant stärkerem Ausmaß von EPCs besiedelt als die Kontrolle, was dessen Eignung zur Herstellung von Gefäßprothesen unterstreicht.

**Biokompatibilitätstests in vivo**

Die Tierversuche wurden von der Tierversuchskommission der Medizinischen Universität Wien und dem österreichischen Bundesministerium für Wissenschaft und Forschung genehmigt. In Beispiel 2 elektrogesponnene Gefäßprothesen (ID: 1,5 mm, Länge: 20 mm), wie in Fig. 2a dargestellt, wurden in die infrarenale Aorta von Inzucht-Ratten der Gattung Sprague Dawley (männlich, Körpergewicht 300-400 g) mikrochirurgisch implantiert (End-zu-End-Anastomosen, Nahtmaterial: Nylon 9/0). Es wurden weder Antikoagulations- noch Thrombozytenaggregationshemmer verabreicht. Die Implantate wurden nach 2 h oder 7 d in Allgemeinnarkose unter Vollheparinisierung der Tiere entnommen und makroskopisch untersucht. Teile der proximalen und distalen Anastomosenregion und der Graftmitte wurden entweder in Formalin oder Glutaraldehyd, 2,5%, präserviert und anschließend histologisch mittels Hämatoxylin-Färbung, Immunhistochemie oder Rasterelektronenmikroskopie untersucht.

Weiters wurde die Thrombozytenadhäsion mittels Mepacrinfärbung untersucht. Dazu wurden die Transplantatstücke nach der Explantation 24 h lang bei 4 °C in 2,5% Glutaraldehyd gelagert. Die Proben wurden dreimal 1 min lang in PBS gewaschen und dann in Mepacrin-Färbelösung (10 mM in destilliertem Wasser, Sigma-Aldrich, USA) 90 min lang bei Raumtemperatur im Dunkeln inkubiert. Die Proben wurden 3-mal in PBS gewaschen, um die restliche Färbelösung zu entfernen, und mit Fluoreszenz-Einbettmedium auf Glas-Objektträgern eingebettet. Anhaftende Thrombozyten wurden mit einem inversen Mikroskop von Olympus betrachtet und aufgenommen.

In den Fig. 11A-H sind die folgenden Fotografien abgebildet:

Fig. 11A die Gefäßprothese und Fig. 10B die luminale Oberfläche der Prothese nach 2 Stunden Implantationszeit;

Fig. 11C eine elektronenmikroskopische Aufnahme der luminalen Oberfläche;

Fig. 11D eine Mepacrin-Färbung der adhärierten Blutplättchen nach 2 Stunden Implantationszeit, wobei kaum adhärierte Thrombozyten sichtbar sind;

Fig. 11E die Prothese direkt nach der Implantation und Fig. 10F die Prothese nach sieben Tagen Implantationszeit;

Fig. 11G die luminale Oberfläche nach sieben Tagen Implantationszeit, wobei keinerlei Thrombosen erkennbar sind; und

Fig. 11H die präparierte Prothese nach einwöchiger Implantation, wobei keinerlei Anzeichen von frühzeitiger Degradation erkennbar und keinerlei Defekte bzw. Aneurysmen sichtbar sind.

All dies belegt die hervorragende Eignung von TPUUs der Formel (I) zur Herstellung von Gefäßprothesen gemäß vorliegender Erfindung.

**Patentansprüche**

1. Verwendung eines thermoplastischen Poly(urethan-harnstoff)-Polyaddukts mit sterisch gehinderten Harnstoffgruppen der nachstehenden Formel (I) in einem Elektrospinn-Verfahren zur Herstellung von Gefäßprothesen:

$$-[I\text{-}M\text{-}(I\text{-}C_1)_a\text{-}(I\text{-}M)_b\text{-}(I\text{-}C_2)_c]_n- \qquad (I)$$

worin I, M, $C_1$ und $C_2$ jeweils für zweiwertige Reste stehen, die jeweils über eine Urethan- oder eine Harnstoffgruppierung miteinander verbunden sind, wovon

I jeweils unabhängig für einen zweiwertigen, von einem Diisocyanat abgeleiteten, gesättigten oder ungesättigten, aliphatischen, alicyclischen oder aromatischen Rest mit 1 bis 20 Kohlenstoffatomen steht;

M jeweils unabhängig für einen zweiwertigen, von einem Makrodiol abgeleiteten Rest eines aliphatischen Polyethers, Polyesters oder Polycarbonats mit einem zahlenmittleren Molekulargewicht $M_n \geq 500$ steht;

$C_1$ jeweils unabhängig für einen zweiwertigen, von einem Diamin oder Aminoalkohol mit jeweils zumindest einer sterisch gehinderten sekundären Aminogruppe durch Entfernung je eines N-gebundenen Wasserstoffatoms des Diamins oder eines N-gebundenen und des O-gebundenen Wasserstoffatoms des Aminoalkohols abgeleiteten, gesättigten oder ungesättigten, aliphatischen oder alicyclischen Rest mit 1 bis 30 Kohlenstoffatomen steht;

$C_2$ jeweils unabhängig für einen zweiwertigen, von einem Diol, Diamin oder Aminoalkohol ohne sterisch gehinderte sekundäre Aminogruppen abgeleiteten, gesättigten oder ungesättigten, aliphatischen, alicyclischen oder aromatischen Rest mit 1 bis 20 Kohlenstoffatomen steht;

wobei in den Resten von I, $C_1$ und $C_2$ bei Vorliegen von mehr als vier Kohlenstoffatomen gegebenenfalls zumindest eines davon durch ein aus Sauerstoff und Stickstoff ausgewähltes Heteroatom ersetzt ist;

wobei zumindest einer der Reste I, M, $C_1$ und $C_2$ eine oder mehrere Estergruppierungen umfasst; und

a, b und c jeweils unabhängig für eine ganze Zahl von 0 bis 10 stehen und n eine Zahl $\geq 3$ ist, die für die Anzahl der Blöcke des Polyaddukts steht;

mit der Maßgabe, dass jeweils innerhalb desselben Blocks $a + c \geq 1$ ist und in allen Blöcken zusammen zumindest ein $a \geq 1$ ist und zumindest ein $c \geq 1$ ist.

2. Verwendung nach Anspruch 1, **dadurch gekennzeichnet, dass** in dem thermoplastischen Poly(urethan-harnstoff)-Polyaddukt zumindest ein Teil der Estergruppierungen unter physiologischen Bedingungen spaltbar ist, die Reste I, M, $C_1$ und $C_2$ sowie etwaige Spaltprodukte davon biokompatibel und physiologisch unbedenklich sind und durch das Elektrospinn-Verfahren eine temporäre Gefäßprothese hergestellt wird.

**3.** Verwendung nach Anspruch 1 oder 2, **dadurch gekennzeichnet, dass** in dem thermoplastischen Poly(urethan-harnstoff)-Polyaddukt:

a und c jeweils unabhängig $\leq 5$ oder $\leq 3$ sind; und/oder
a und c jeweils unabhängig $\geq 1$ sind; und/oder
$b \geq 1$ ist; und/oder
$b = c$ oder $b = a$ oder $b + 1 = a + c$ ist; und/oder
$n \geq 5$ oder $n \geq 10$ oder $n \geq 50$ ist.

**4.** Verwendung nach einem der Ansprüche 1 bis 3, **dadurch gekennzeichnet, dass** in dem thermoplastischen Poly(urethan-harnstoff)-Polyaddukt:

die Reste I jeweils unabhängig von einem Diisocyanat aus der aus den folgenden bestehenden Gruppe abgeleitet sind: 1,6-Hexamethylendiisocyanat, 4,4'-Diisocyanatodicyclohexylmethan, Isophorondiisocyanat, 1,3-Bis(isocyanatomethyl)cyclohexan, Diphenylmethan-4,4'-diisocyanat, L-Lysinethylesterdiisocyanat; und/oder
die Reste M jeweils unabhängig von einem Polyether, Polyester oder Polycarbonat aus der aus den folgenden bestehenden Gruppe abgeleitet sind: Polytetrahydrofuran, Polyethylenglykol, Polypropylenglykol, Polycaprolacton, Polylactid, Polyglykolid, Poly(lactid-co-glykolid), Polyhexamethylencarbonat.

**5.** Verwendung nach einem der Ansprüche 1 bis 4, **dadurch gekennzeichnet, dass** in dem thermoplastischen Poly(urethan-harnstoff)-Polyaddukt die Reste $C_1$ jeweils unabhängig von einem Diamin abgeleitet und aus Resten der nachstehenden Formel (II) ausgewählt sind:

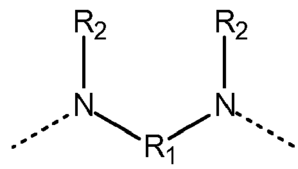

(II)

worin die gestrichelten Linien jeweils die Bindung an die Carbonylgruppe einer die Reste I, M, $C_1$ und $C_2$ verbindenden Urethan- oder Harnstoffgruppierung anzeigen;
$R_1$ aus zweiwertigen, gesättigten oder ungesättigten, aliphatischen oder alicyclischen Resten mit 1 bis 20 Kohlenstoffatomen ausgewählt ist; und
die $R_2$ jeweils unabhängig aus Wasserstoff sowie einwertigen, sperrigen, gesättigten oder ungesättigten, aliphatischen oder alicyclischen Resten mit 1 bis 10 Kohlenstoffatomen ausgewählt sind, mit der Maßgabe, dass nicht beide $R_2$ gleichzeitig Wasserstoff sind.

**6.** Verwendung nach Anspruch 5, **dadurch gekennzeichnet, dass** in dem thermoplastischen Poly(urethan-harnstoff)-Polyaddukt:

$R_1$ aus $C_1$-$C_{10}$-Alkylen- und $C_4$-$C_{10}$-Cycloalkylenresten ausgewählt ist oder aus $C_2$-$C_6$-Alkylen- und $C_5$-$C_6$-Cycloalkylenresten ausgewählt ist; und/oder
die $R_2$ jeweils unabhängig aus 1,1-Dimethyl-substituierten, gesättigen oder ungesättigten $C_1$-$C_6$-Alkylresten und 1-Methyl-substituierten $C_3$-$C_6$-Cycloalkylresten ausgewählt sind oder aus Isopropyl, tert-Butyl, 1,1 -Dimethylpropyl und 1-Methylcyclohexyl ausgewählt sind.

**7.** Verwendung nach einem der Ansprüche 1 bis 6, **dadurch gekennzeichnet, dass** in dem thermoplastischen Poly(urethan-harnstoff)-Polyaddukt zumindest einer der Reste $C_2$ eine oder mehrere Estergruppierungen umfasst, die gegebenenfalls jeweils unabhängig von einem Diol aus der aus den folgenden bestehenden Gruppe abgeleitet sind: Bis(hydroxyethyl)terephthalat, Bis(hydroxypropyl)carbonat, 2-Hydroxyethyllactat und 2-Hydroxyethylglykolat.

8. Verwendung nach einem der Ansprüche 1 bis 7, **dadurch gekennzeichnet, dass** in dem thermoplastisches Poly(urethan-harnstoff)-Polyaddukt b + 1 = a + c ist und das Polyaddukt der nachstehenden Formel (IV) entspricht:

$$-[(I\text{-}M\text{-}I\text{-}C_1)_a\text{-}(I\text{-}M\text{-}I\text{-}C_2)_c]_n\text{-} \qquad (IV)$$

worin

a und c jeweils unabhängig aus 1 bis 3 ausgewählt sind oder
a und cjeweils 1 sind; und
$n \geq 5$ oder $n \geq 10$ oder $n \geq 20$ ist.

9. Verwendung nach einem der Ansprüche 1 bis 8, **dadurch gekennzeichnet, dass** in dem Elektrospinn-Verfahren eine Lösung des TPUU der Formel (I) in einem organischen Lösungsmittel oder Lösungsmittelgemisch in einer Elektrospinnvorrichtung, die einen Hochspannungsgenerator, eine Spritzenpumpe, eine Spritze mit stumpfem Ende als Elektrode, einen geerdeten, elektrisch leitenden rotierenden Stahldorn als Kollektorelektrode und gegebenenfalls eine Hilfselektrode umfasst, mittels der Spritze in das zwischen den Elektroden aufgebaute elektrische Feld injiziert und die sich dabei bildenden Polymerfasern als Endlos-Nanofasern auf dem rotierenden Dorn zu einer als Gefäßprothese geeigneten Röhre aufgewickelt werden.

10. Verwendung nach Anspruch 9, **dadurch gekennzeichnet, dass** eine Lösung des TPUU der Formel (I) in Hexafluorisopropanol eingesetzt wird.

11. Verwendung nach Anspruch 9 oder 10, **dadurch gekennzeichnet, dass** eine Lösung eines Gemischs aus dem TPUU der Formel (I) und zumindest einem weiteren Polymer eingesetzt wird und aus dem Gemisch bestehende Röhren hergestellt werden.

12. Verwendung nach Anspruch 11, **dadurch gekennzeichnet, dass** das zumindest eine weitere Polymer in einem Anteil von zumindest 10 Gew.-%, zumindest 30 Gew.-% oder zumindest 50 Gew.-% des Gemischs eingesetzt wird.

13. Verwendung nach Anspruch 11 oder 12, **dadurch gekennzeichnet, dass** ein Gemisch aus dem TPUU der Formel (I) und einem biologisch abbaubaren TPU eingesetzt wird.

14. Verwendung nach Anspruch 13, **dadurch gekennzeichnet, dass** als biologisch abbaubares TPU ein Polyetherurethan, gegebenenfalls ein Polyaddukt aus Polytetrahydrofuran, Bis(hydroxyethyl)terephthalat und Hexamethylendiisocyanat, im Gemisch eingesetzt wird.

15. Gefäßprothese, die durch ein Elektrospinn-Verfahren unter Verwendung von thermoplastischen Poly(urethan-harnstoff)-Polyaddukten der Formel (I) nach einem der Ansprüche 1 bis 14 erhältlich ist.

**Beispiel 1**

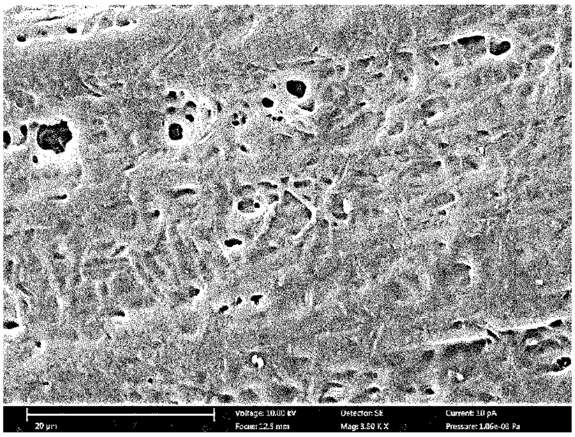

**Fig. 1a**
(innen)

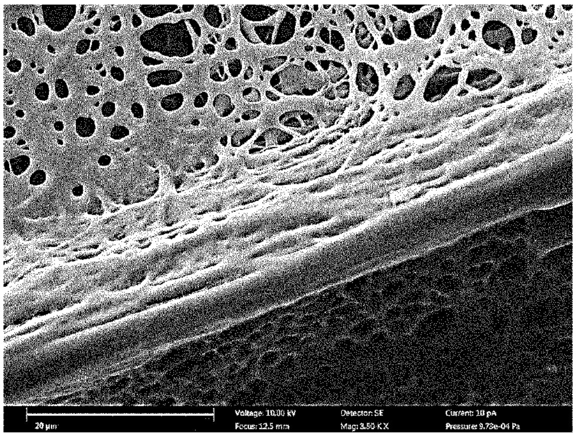

**Fig. 1b**
(außen)

## Figur 1

**Beispiel 2**

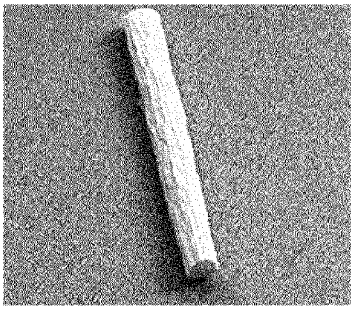

**Fig. 2a**
(außen)

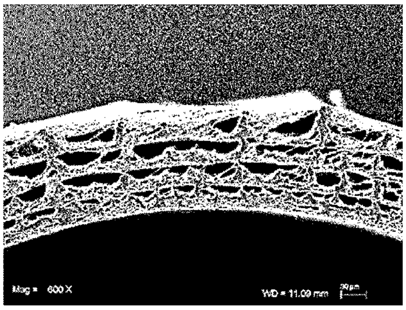


**Fig. 2b**
(Querschnitt)

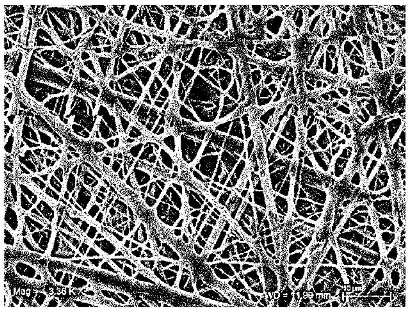

**Fig. 2c**
(innen)

## Figur 2

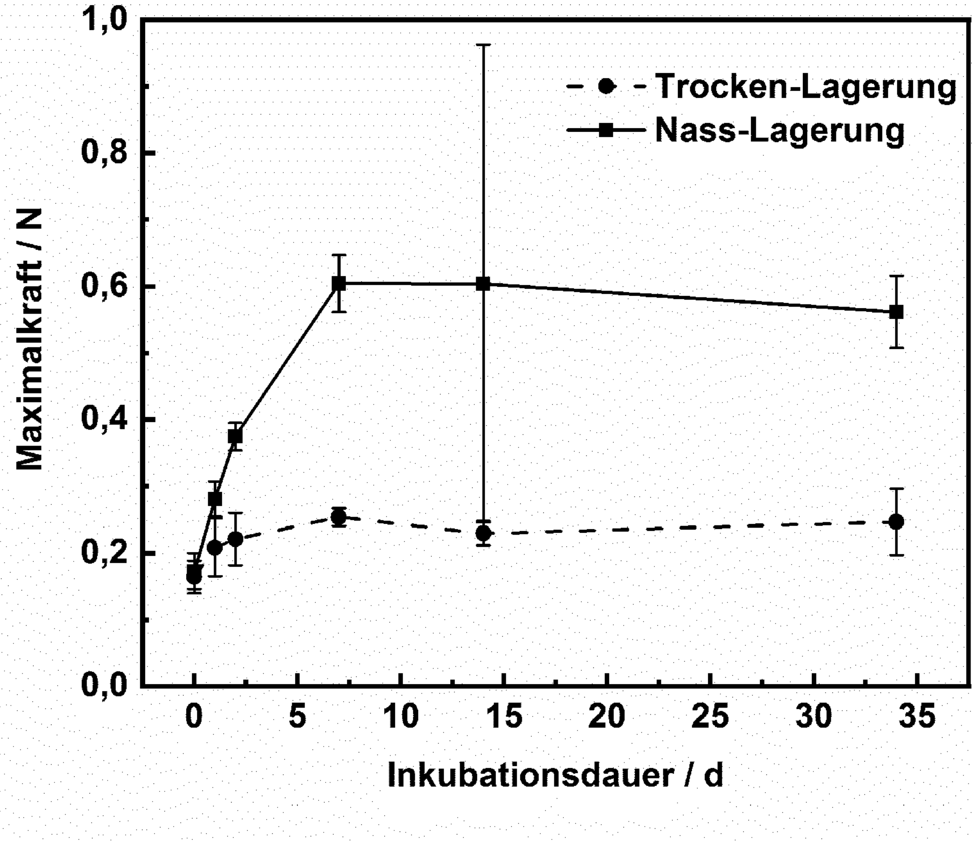
1,0
0,8
0,6
0,4
0,2
0,0
Maximalkraft / N
Trocken-Lagerung
Nass-Lagerung
0
5
10
15
20
25
30
35
Inkubationsdauer / d

Figur 3

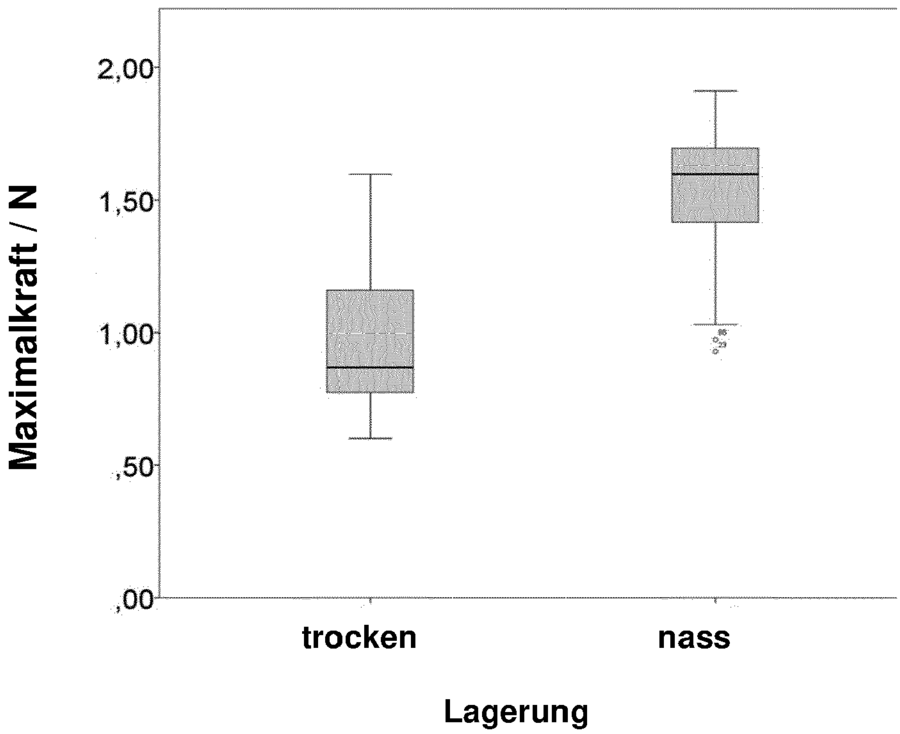


Figur 4

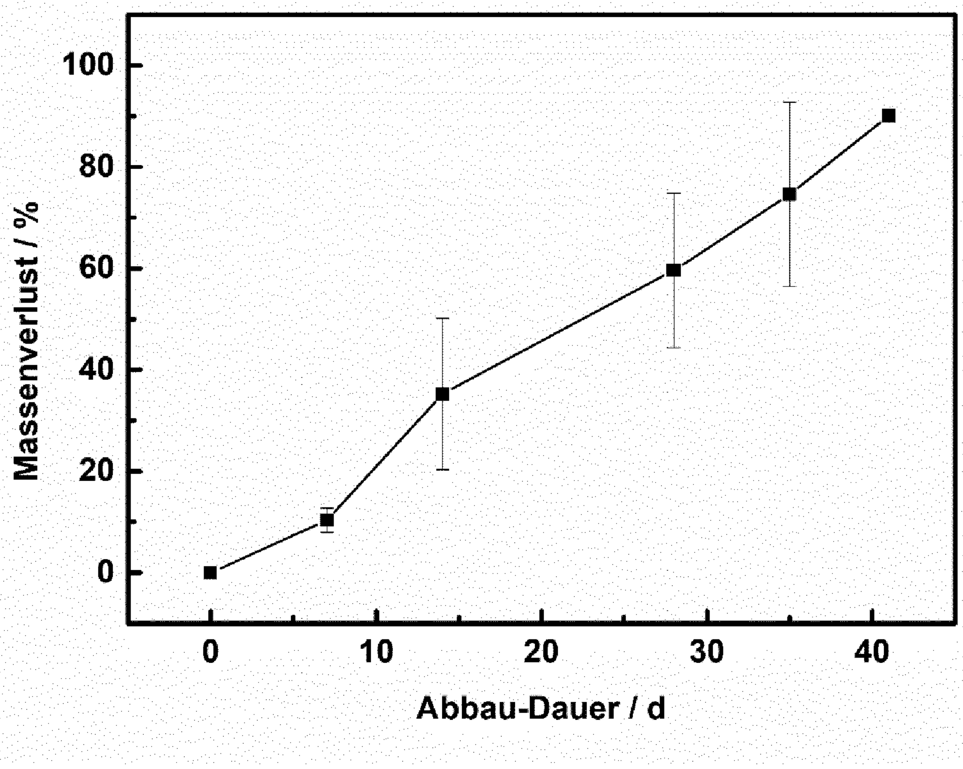


Figur 5

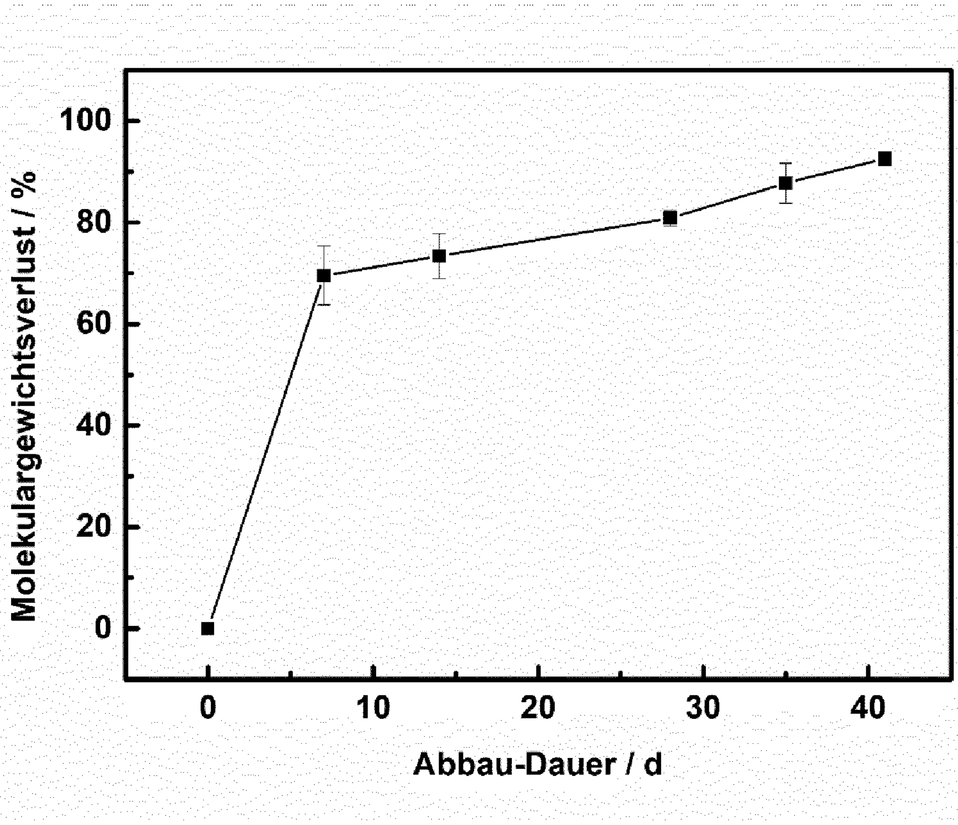
100
80
60
40
20
0
Molekulargewichtsverlust / %
0
10
20
30
40
Abbau-Dauer / d

Figur 6

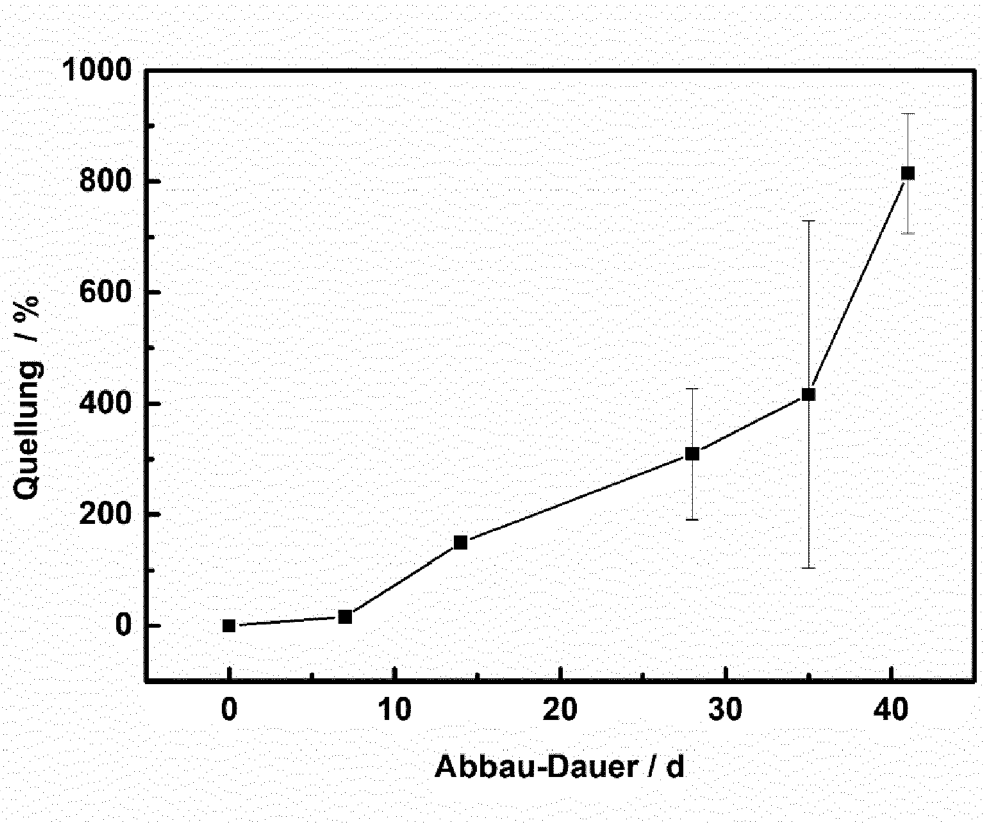


Figur 7

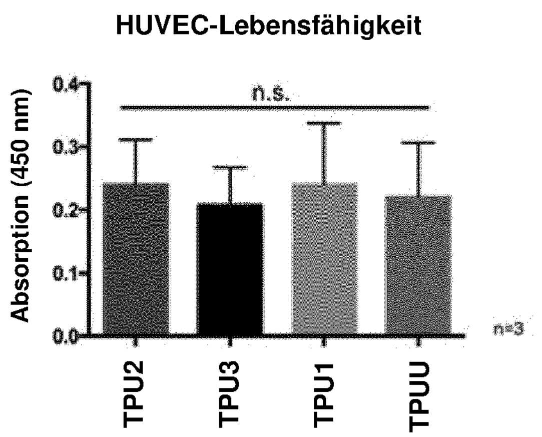
HUVEC-Lebensfähigkeit
Absorption (450 nm)
0.4
0.3
0.2
0.1
0.0
n.s.
n=3
TPU2
TPU3
TPU1
TPUU

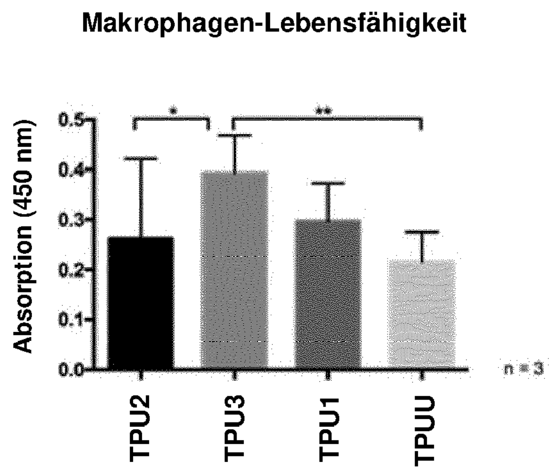
Makrophagen-Lebensfähigkeit
Absorption (450 nm)
0.5
0.4
0.3
0.2
0.1
0.0
*
**
n = 3
TPU2
TPU3
TPU1
TPUU

Figur 8

HUVEC-Lebensfähigkeit in Gegenwart von TBEDA

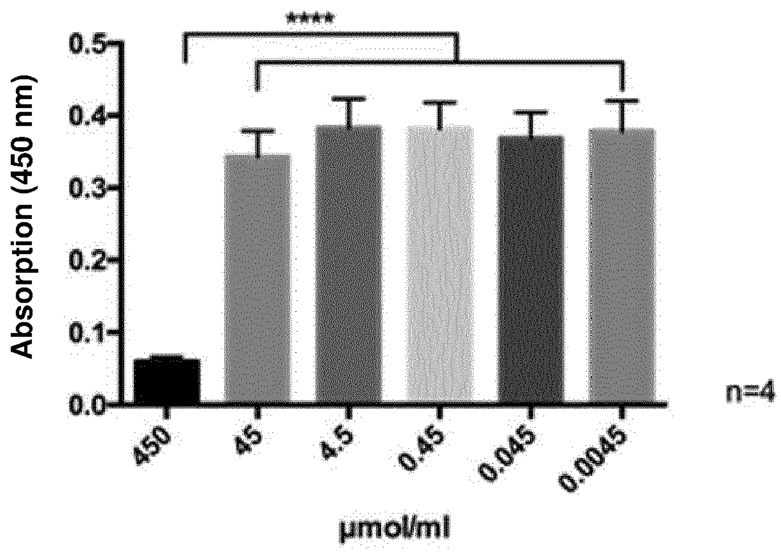


Figur 9

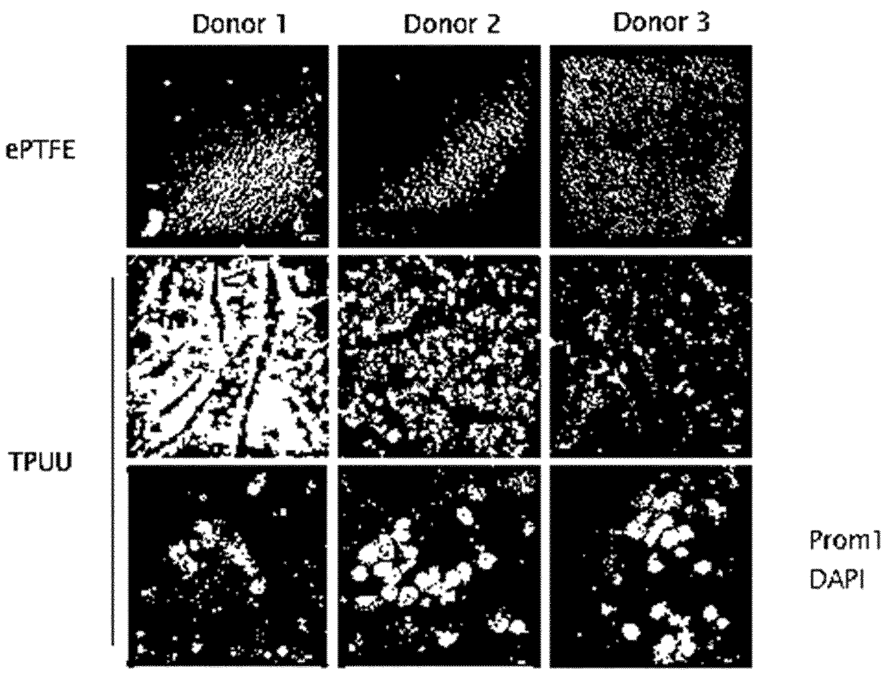


Figur 10

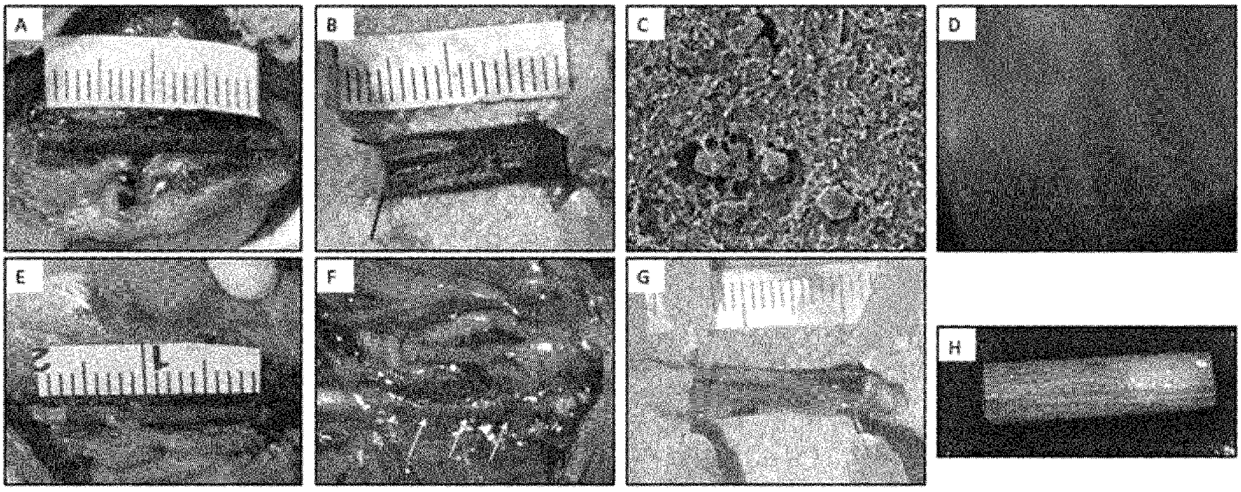


Figur 11

Europäisches Patentamt
European Patent Office
Office européen des brevets

# EUROPÄISCHER RECHERCHENBERICHT

Nummer der Anmeldung
EP 21 15 3995

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br>Y<br>A | US 2020/108550 A1 (CHEN KAI [US] ET AL) 9. April 2020 (2020-04-09)<br>* Absätze [0002], [0010], [0302], [0368]; Ansprüche *<br>* Absatz [0250] - Absatz [0285]; Beispiele 1-17, 18-61; Tabellen 1, 2 *<br>----- | 15<br><br>1-12,15<br>13,14 | INV.<br>C08G18/24<br>C08G18/48<br>C08G18/73<br>C08G18/32<br>C08G18/10<br>A61F2/00<br>C08L75/02<br>A61F2/06 |
| A | EP 1 729 783 A1 (COMMW SCIENT IND RES ORG [AU]) 13. Dezember 2006 (2006-12-13)<br>* Absätze [0001], [0018], [0019]; Ansprüche 1-13 *<br>* Beispiele *<br>----- | 1-15 | |
| Y<br><br>A | US 2017/340463 A1 (CHUN YOUNGJAE [US] ET AL) 30. November 2017 (2017-11-30)<br>* Absätze [0002], [0029] - Absatz [0038]; Ansprüche 1-15 *<br>* Absatz [0068]; Beispiele *<br>----- | 1-12,15<br><br>13,14 | |
| A | PALUMBO R ET AL: "Der arteriovenöse Zugang mit biologischem Material; Langzeitergebnisse der Omniflow-II-Prothese für die Dialyse", GEFÄSSCHIRURGIE ; ZEITSCHRIFT FÜR VASKULÄRE UND ENDOVASKULÄRE CHIRURGIE, SPRINGER, BERLIN, DE,<br>Bd. 15, Nr. 2, 6. März 2010 (2010-03-06), Seiten 108-112, XP019805544,<br>ISSN: 1434-3932<br>* Titel *<br>* Seite 108, mittlere Spalte, Zeile 5 - rechte Spalte, Zeile 3 *<br>* Referenzen [2, 3];<br>Seite 112, linke Spalte *<br>-----<br>-/-- | 1-15 | RECHERCHIERTE SACHGEBIETE (IPC)<br>C08G<br>A61F<br>C08L |

2

Der vorliegende Recherchenbericht wurde für alle Patentansprüche erstellt

| Recherchenort | Abschlußdatum der Recherche | Prüfer |
|---|---|---|
| München | 6. Juli 2021 | Paulus, Florian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 21 15 3995

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 5 955 560 A (TANZI MARIA CRISTINA [IT]) 21. September 1999 (1999-09-21)<br>* Ansprüche 1-15 *<br>* Spalte 1, Zeile 5 - Zeile 10 *<br>* Spalte 6, Zeile 42 - Spalte 7, Zeile 7 *<br>----- | 1-15 | |
| Y<br><br><br><br><br><br><br><br><br>A | BERGMEISTER HELGA ET AL: "Biodegradable, thermoplastic polyurethane grafts for small diameter vascular replacements",<br>ACTA BIOMATERIALIA,<br>Bd. 11, 10. September 2014 (2014-09-10), Seiten 104-113, XP055821172,<br>Amsterdam , NL<br>ISSN: 1742-7061, DOI: 10.1016/j.actbio.2014.09.003<br>* Titel; Zusammenfassung *<br>* 2.1 Polymer synthesis; 2.2 Graft fabrication *<br>* 3. Results *<br>----- | 1-12,15<br><br><br><br><br><br><br><br><br>13,14 | |
| | | | RECHERCHIERTE SACHGEBIETE (IPC) |

Der vorliegende Recherchenbericht wurde für alle Patentansprüche erstellt

2

| Recherchenort | Abschlußdatum der Recherche | Prüfer |
|---|---|---|
| München | 6. Juli 2021 | Paulus, Florian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................
& : Mitglied der gleichen Patentfamilie, übereinstimmendes Dokument

EPO FORM 1503 03.82 (P04C03)

Seite 2 von 2

## ANHANG ZUM EUROPÄISCHEN RECHERCHENBERICHT ÜBER DIE EUROPÄISCHE PATENTANMELDUNG NR.

EP 21 15 3995

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-07-2021

| Im Recherchenbericht angeführtes Patentdokument | Datum der Veröffentlichung | Mitglied(er) der Patentfamilie | Datum der Veröffentlichung |
|---|---|---|---|
| US 2020108550 A1 | 09-04-2020 | US 2018361660 A1 | 20-12-2018 |
| | | US 2020108550 A1 | 09-04-2020 |
| | | WO 2017112571 A1 | 29-06-2017 |
| EP 1729783 A1 | 13-12-2006 | CN 1950098 A | 18-04-2007 |
| | | EP 1729783 A1 | 13-12-2006 |
| | | JP 5496457 B2 | 21-05-2014 |
| | | JP 2007530101 A | 01-11-2007 |
| | | MY 146259 A | 31-07-2012 |
| | | TW 200604249 A | 01-02-2006 |
| | | US 2006051394 A1 | 09-03-2006 |
| | | US 2015246994 A1 | 03-09-2015 |
| | | WO 2005089778 A1 | 29-09-2005 |
| US 2017340463 A1 | 30-11-2017 | US 2017340463 A1 | 30-11-2017 |
| | | WO 2016077410 A1 | 19-05-2016 |
| US 5955560 A | 21-09-1999 | DE 69807556 T2 | 30-04-2003 |
| | | EP 0870766 A1 | 14-10-1998 |
| | | ES 2183249 T3 | 16-03-2003 |
| | | IT MI970802 A1 | 08-10-1998 |
| | | US 5955560 A | 21-09-1999 |
| | | US 6143893 A | 07-11-2000 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 452775 A2
- WO 2014144539 A2
- WO 2016069582 A1
- WO 2016126103 A1
- WO 2016126756 A1
- WO 2017155958 A1
- EP 20183957 A


### In der Beschreibung aufgeführte Nicht-Patentliteratur


- **BAUDIS S. ; LIGON S. C. ; SEIDLER K. ; WEIGEL G. ; GRASL C. ; BERGMEISTER H. ; SCHIMA H. ; LISKA R.** Hard-block degradable thermoplastic urethane-elastomers for electrospun vascular prostheses. *J. Polym. Sci. A,* 2012, vol. 1 (50), 1272-1280
- **SEIDLER K. ; EHRMANN K. ; STEINBAUER P. ; ROHATSCHEK A. ; ANDRIOTIS O. G. ; DWORAK C. ; KOCH T. ; BERGMEISTER H. ; GRASL C. ; SCHIMA H.** A structural reconsideration: Limearalphatic or alicyclic hard segments for biodegradable thermoplastic polyurethanes?. *J. Polym. Sci. A,* 2018, vol. 1 (56), 2214-2224
- **EHRMANN K. ; POTZMANN P. ; DWORAK C. ; BERGMEISTER H. ; EILENBERG M. ; GRASL C. ; KOCH T. ; SCHIMA H. ; LISKA R. ; BAUDIS S.** Hard Block Degradable Polycarbonate Urethanes: Promising Biomaterials for Electrospun Vascular Prostheses. *Biomacromolecules,* 2020, vol. 21 (2), 376-387
- **STOWELL, J. C. ; PADEGIMAS, S. J.** Urea dissociation. Measure of steric hindrance in secondary amines. *J. Org. Chem.,* 1974, vol. 39 (16), 2448-2449
- **HUTCHBY, M. ; HOULDEN, C. E. ; FORD, J. G. ; TYLER, S. N. G. ; GAGNE, M. R. ; LLOYD-JONES, G. C. ; BOOKER-MILBURN, K. I.** Hindered ureas as masked isocyanates: facile carbamoylation of nucleophiles under neutral conditions. *Angew. Chem. Int. Ed.,* 2009, vol. 48 (46), 8721-8724
- **YING, H. ; ZHANG, Y. ; CHENG, J.** Dynamic urea bond for the design of reversible and self-healing polymers. *Nat. Commun.,* 2014, vol. 5, 3218
- **ZHANG, Y. ; YING, H. ; HART, K. R. ; WU, Y. ; HSU, A. J. ; COPPOLA, A. M. ; KIM, T. A. ; YANG, K. ; SOTTOS, N. R. ; WHITE, S. R.** Malleable and recyclable poly-(urea-urethane) thermosets bearing hindered urea bonds. *Adv. Mater.,* 2016, vol. 28 (35), 7646-7651
- **ZHANG, L. ; ROWAN, S. J.** Effect of sterics and degree of cross-linking on the mechanical properties of dynamic poly(alkylurea-urethane) networks. *Macromolecules,* 2017, vol. 50 (13), 5051-5060
- **BRUCE, A. C. ; LEWIS, C. L.** Influence of glass transition temperature on mechanical and self-healing behavior of polymers bearing hindered urea bonds. *SPE ANTEC,* 2017
- **FANG, Z. ; ZHENG, N. ; ZHAO, Q. ; XIE, T.** Healable, reconfigurable, reprocessable thermoset shape memory polymer with highly tunable topological rearrangement kinetics. *ACS Appl. Mater. Interfaces,* 2017, vol. 9 (27), 22077-22082
- **WANG, Y. ; PAN, Y. ; ZHENG, Z. ; DING, X.** Reconfigurable and reprocessable thermoset shape memory polymer with synergetic triple dynamic covalent bonds. *Macromol. Rapid Commun.,* 2018, vol. 39 (10), 1800128
- **YING, H. ; CHENG, J.** Hydrolyzable polyureas bearing hindered urea bonds. *J. Am. Chem. Soc.,* 2014, vol. 136 (49), 16974-16977
- **YING, H. ; YEN, J. ; WANG, R. ; LAI, Y. ; HSU, J.-L.-A. ; HU, Y. ; CHENG, J.** Degradable and biocompatible hydrogels bearing a hindered urea bond. *Biomater. Sci.,* 2017, vol. 5 (12), 2398-2402
- **CAI, K. ; YING, H. ; CHENG, J.** Dynamic Ureas with fast and pHindependent hydrolytic kinetics. *Chem. Eur. J.,* 2018, vol. 24 (29), 7345-7348
- **CHEN, M. ; FENG, X. ; XU, W. ; WANG, Y. ; YANG, Y. ; JIANG, Z. ; DING, J.** PEGylated polyurea bearing hindered urea bond for drug delivery. *Molecules,* 2019, vol. 24 (8), 1538